# EUROPEAN PATENT SPECIFICATION

(11) **EP 4 566 586 B1**
(45) Date of publication and mention of the grant of the patent: **21.10.2026**
(21) Application number: 23848132.9
(22) Date of filing: 11.04.2023
(51) Int. Cl.: A61P 17/06, A61P 17/10, A61Q 1/14, A61P 31/10, A61P 17/04, A61Q 19/02, A61Q 19/08, A61Q 19/10, A61Q 17/04, A61K 31/203, A61P 17/02, A61K 9/00, A61K 9/06, A61K 31/19, A61P 17/00, A61K 47/20, A61K 8/46, A61K 31/185, A61K 31/60, A61K 31/20, A61K 31/045

(54) **SKIN-CARE PRODUCT COMPOSITION AND PHARMACEUTICAL COMPOSITION COMPRISING SULFONATED CALIXARENE, AND USE OF SULFONATED CALIXARENE**
HAUTPFLEGEPRODUKTZUSAMMENSETZUNG UND PHARMAZEUTISCHE ZUSAMMENSETZUNG MIT SULFONIERTEM CALIXAREN UND VERWENDUNG VON SULFONIERTEM CALIXAREN
COMPOSITION DE PRODUIT DE SOIN DE LA PEAU ET COMPOSITION PHARMACEUTIQUE COMPRENANT DU CALIXARÈNE SULFONÉ, ET UTILISATION DE CALIXARÈNE SULFONÉ

(30) Priority: 03.08.2022 CN 202210928325
(43) Date of publication of application: 11.06.2025
(73) Proprietor: Calix Biotechnology (Hangzhou) Co., Ltd, Xiaoshan District Hangzhou, Zhejiang 311200 (CN)
(72) Inventor: GUO, Dongsheng, Tianjin 300071 (CN); LI, Shihui, Tianjin 300071 (CN); WANG, Honglei, Suzhou, Jiangsu 215000 (CN)
(74) Representative: EIP
(86) International application number: PCT/CN2023/087475
(87) International publication number: WO 2024/027193

(56) References cited:
- WO-A1-2016/152954
- CN-A- 1 265 024
- CN-A- 102 697 666
- CN-A- 104 211 970
- CN-A- 114 159 324
- CN-A- 114 302 885
- US-A1- 2010 292 272
- US-A1- 2011 281 948
- US-A1- 2017 145 351
- OZYILMAZ ELIF ET AL: "Inclusion complexes of water-soluble calix[n]arenes with quercetin: preparation, characterization, water solubility, and antioxidant features", MONATSHEFTE FÜR CHEMIE - CHEMICAL MONTHLY, SPRINGER VIENNA, VIENNA, vol. 153, no. 2, 10 January 2022 (2022-01-10), pages 201 - 209, XP037698277, ISSN: 0026-9247, [retrieved on 20220110], DOI: 10.1007/S00706-021-02885-6
- PASHIROVA TATIANA N ET AL: "Supramolecular systems based on calix[4]resorcine with mono-, di-, and tetracationic surfactants: Synergetic structural and solubilization behavior", COLLOIDS AND SURFACES A : PHYSIOCHEMICAL AND ENGINEERINGS ASPECTS, ELSEVIER, AMSTERDAM, NL, vol. 448, 13 February 2014 (2014-02-13), pages 67 - 72, XP028631337, ISSN: 0927-7757, DOI: 10.1016/J.COLSURFA.2014.02.012
- YI WONG YEONG ET AL: "Calixarenes as Host Molecules for Drug Carriers in the Cosmetic and Medical Field", MACROMOLECULAR RESEARCH, vol. 30, no. 12, 6 October 2022 (2022-10-06), Seoul, pages 853 - 862, XP093326242, ISSN: 1598-5032, DOI: 10.1007/s13233-022-0094-9
- ZHOU YUNYOU, DING XUEPING, FANG XIAOLI, LI TAO, TANG DONGBAO, LU QIN: "Studies on the Inclusion Behavior of Amphiphilic p-Sulfonatocalix[4]arene with Ascorbic Acid by Spectrofluorometric Titrations", OPTICS AND PHOTONICS JOURNAL, vol. 01, no. 02, 1 January 2011 (2011-01-01), pages 59 - 64, XP093136594, ISSN: 2160-8881, DOI: 10.4236/opj.2011.12009
- HALL, IRIS H. ET AL.: "Anti-inflammatory Activity of (Polyphenolic)-Sulfonates and Their Sodium Salts in Rodents", METAL-BASED DRUGS, vol. 5, no. 2, 1 January 1998 (1998-01-01), XP055841878, ISSN: 0793-0291, DOI: 10.1155/MBD.1998.67

## Description

### Field of the Invention

The invention belongs to the field of skincare products, specifically involving a skin-care product composition and a pharmaceutical composition, comprising sulfonated calixarene supramolecular compounds as excipients, as well as new uses of sulfonated calixarene supramolecular compounds in skincare and the treatment of skin inflammatory diseases.

### Background of the Invention

Skincare products are daily-use cosmetics that can supplement skin nutrients, moisturize and lock in water, regulate the skin's oil-water balance, promote healthy and moisturized skin, and achieve beautifying effects. They also have functions such as anti-wrinkle, anti-aging, anti-acne, whitening, anti-inflammatory, and soothing sensitivity (Liu Zhihong et al., China Food Drug Administration, 2020, (05), 98-101+126). Medical skincare products do not have a strict definition in China. They are also referred to as "functional cosmetics" or "cosmeceuticals" abroad. They were proposed by Albert Kligman in 1984 as a third category of products between cosmetics and medicines (Albert Kligman et al., Dermatologic Surgery. 2005, 31 (7 Pt 2): 890-891). In essence, they are cosmetics, not pharmaceuticals. Functional skincare products are increasingly favored and can be divided into several categories: cleansing, moisturizing and restoring skin barrier, oil control, anti-inflammatory, soothing, sun protection, whitening and freckle removal. Additionally, the application of medical cosmetics in skin diseases such as dermatitis and eczema is also increasing (Gou Weijun et al., Fine and Specialty Chemicals, 2020, 28 (09), 1-5). To achieve good skincare effects, these active ingredients in cosmetics need to have certain stability and should not be irritating.

In recent years, the incidence of skin diseases has been relatively high, especially diseases closely related to skincare products, such as acne, sensitive facial skin, hormone-dependent dermatitis, perioral dermatitis, rosacea, and chloasma, are quite common (Zhao Hengguang et al., Dermatology Bulletin, 2017, 34(04), 462-467+9). These diseases almost invariably face the current situation of using topical drugs in clinical practice. Under this premise, how to choose skincare products and how to coordinate the use of skincare products and topical drugs at the same time are issues that must be carefully solved in skincare consultation (Qi Xianlong, et al., Chinese Journal of Aesthetic Medicine, 2009, 18(02): 240-241; Dreno B, et al., J Eur Acad Dermatol Venereol, 2014, 28 (11): 1409-1417). The reasonable use of skincare products can not only play a role in repairing the skin barrier and relieving inflammation but also alleviate symptoms such as skin dryness, burning, and itching, reduce drug dosage, prevent the recurrence of skin diseases, and improve the quality of life of patients (Li Li, et al., The Chinese Journal of Dermatovenereology, 2015, 29(06): 553-555; He Li, et al., Journal of Clinical Dermatology, 2009, 38(6): 409-410; Willis CM, et al., Br J Dermatol, 2001, 145 (2):258-263). For skin diseases such as skin barrier damage and seborrhea, soothing, cleansing, moisturizing and skin barrier repairing skincare products are often chosen (Zhou Xiaotong et al., Journal of Practical Dermatology, 2016, 9(03), 175-179; Lodén M et al., Clin Dermatol, 2012, 30(3):286-296).

With the continuous upgrading of consumer demand, the development of multi-functional skincare products has become a trend. When adding multiple skincare active ingredients into the same skincare product, the problem of preventing the active ingredients from interfering with each other must be solved. Furthermore, the addition concentration and stability of active ingredients are also very important for their effects, and the permeability and irritation of active ingredients on the skin need to be carefully examined. It is an increasing demand in the field of skincare products to comprehensively consider the above-mentioned factors, optimize the preparation process from multiple aspects, optimize the carrier and dosage form of the active ingredients, and thus prepare skincare products with excellent effects. Taking vitamin A, a widely used anti-aging ingredient on the market, as an example, its poor solubility in water means it can only be dissolved in oily matrices, which may give consumers a greasy and uncomfortable feeling. In addition, vitamin A is unstable to light, heat, oxygen, and water, so the concentration of vitamin A used in skincare products is likely to be lower than the initial added concentration. As another example, retinoic acid, a medically used ingredient for treating acne, has poor solubility in water and thus can only be dissolved in oily matrices; it is also unstable in the presence of light, heat, and water; furthermore, retinoic acid is irritating, causing patients a painful experience during use. Therefore, there is an urgent need for a suitable delivery platform for skincare active ingredients to solve the solubility and stability of these active ingredients while improving their transdermal permeability, reducing irritation, and ensuring that multiple cosmetic ingredients do not interfere with each other, and even providing synergistic effects.

### Summary of the Invention

A purpose of the invention is to provide a molecular container platform for encapsulating skincare active substances, thereby improving the water solubility, stability and effectiveness of the skincare active substances and reducing irritation.

More specifically, the invention provides a skin-care product composition comprising at least one skincare active substance and a sulfonated calixarene compound of formula (I): 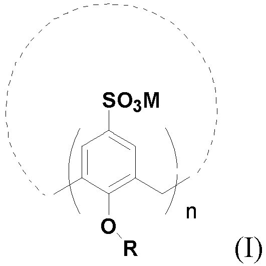 wherein,
n is an integer selected from 4 to 8,
M is independently selected from H, alkali metals, and alkaline earth metals,
R is independently selected from C₄₋₁₆ straight-chain alkyl.

In preferred embodiments, M is at least one metal selected from the group consisting of Na, K, Mg, and Ca, more preferably Na.

In other preferred embodiments, R is selected from C₈₋₁₂ straight-chain alkyl, more preferably dodecyl.

In preferred embodiments, the at least one skincare active substance is one or more active substances selected from basic skincare products or functional skincare products, especially active substances with at least one function of cleansing or makeup removal, whitening, anti-aging (such as anti-wrinkle, antioxidant), anti-inflammatory or anti-acne, moisturizing, and sun protection.

Preferably, the active substances with cleansing or makeup removal functions are selected from: polyols such as butylene glycol, polyethylene glycol, and dipropylene glycol, surfactants such as sodium dodecyl sulfate, sodium laureth sulfate, sodium acyl sulfate, decyl glucoside, cocamidopropyl betaine, and amino acids, and synthetic esters such as isopropyl myristate, isopropyl hexadecanoate, and triglyceride.

The active substances with whitening functions are selected from: vitamin C and derivatives thereof (such as sodium/magnesium ascorbyl phosphate, ascorbyl glucoside, ethyl ascorbic acid, and ascorbyl tetraisopalmitate), phenylethyl resorcinol, niacinamide, tranexamic acid, arbutin, ellagic acid, p-dihydroxybenzene, kojic acid, glutathione, salicylic acid, tretinoin, and hydroquinone.

The active substances with anti-aging functions are selected from: vitamin A, proanthocyanidins, vitamin E, resveratrol, peptides (such as hexapeptide, palmitoyl tripeptide-5), bifida ferment lysate, astaxanthin, epidermal growth factor, glycolic acid and lactic acid, ubiquinone, caffeine, glycolic acid, lactic acid, vitamin C and derivatives thereof.

The active substances with anti-inflammatory or anti-acne functions are selected from: retinoic acid, α-hydroxy acid, salicylic acid, azelaic acid, and α-bisabolol.

The active substances with moisturizing functions are selected from: ceramides, sphingolipids, phospholipids, cholesterol, lecithin, squalane, hyaluronic acid, chondroitin sulfate, natural moisturizing factor, sorbitol, mannitol, glucose, trehalose, glycerin, pentylene glycol, butylene glycol, and provitamin B5.

The active substances with sun protection functions are selected from: benzophenone-3, octyl methoxycinnamate, ensulizole, PEG-25 p-aminobenzoic acid, ethylhexyl triazone, 2-ethylhexyl 2-cyano-3,3-diphenylacrylate, pentyl Padimate-O, polyacrylamide methylbenzylidene camphor, glyceryl p-aminobenzoate, diethylamino hydroxybenzoyl hexyl benzoate, camphor benzalkonium methosulfate, benzophenone-5, homosalate, ethylhexyl methoxycinnamate, isoamyl methoxycinnamate, and disodium phenyl dibenzimidazole tetrakisulfonate.

The inventors have found that calixarenes, as macrocyclic host molecules, can encapsulate skincare active substances through host-guest interaction, thereby serving to protect the aforementioned active substances. Applying calixarenes to the field of skincare, new supramolecular skincare formulations can be developed to meet the demand for more effective skin improvement. Therefore, not only can calixarenes be used to enhance the stability and water solubility of skincare active substances, but they can also be used to encapsulate multiple functional molecules simultaneously to protect the functions of each molecule from interfering with or affecting each other. Host-guest interaction is an important basis for applying calixarenes to the development of new supramolecular skincare active ingredients. Calixarenes exhibit strong selective binding to a variety of bioactive substances, which will have a broad market application in the field of skincare.

Another purpose of the invention is to provide a molecular container platform for encapsulating anti-inflammatory active substances, which can be used to prepare pharmaceutical compositions for treating skin inflammatory diseases. This molecular container can improve the water solubility, stability, and effectiveness of active substances and reduce the irritation and side effects of anti-inflammatory active substances.

More specifically, the invention provides a pharmaceutical composition for treating skin inflammatory diseases, comprising at least one active substance with skin anti-inflammatory function and a sulfonated calixarene compound of formula (I): 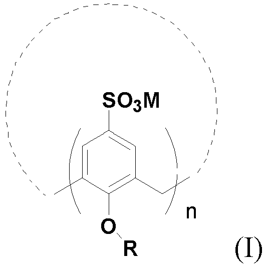 wherein,
n is an integer selected from 4 to 8,
M is independently selected from H, alkali metals, and alkaline earth metals
R is independently selected from C₄₋₁₆ straight-chain alkyl.

In preferred embodiments, M is at least one metal selected from the group consisting of Na, K, Mg, and Ca, more preferably Na.

In other preferred embodiments, R is selected from C₈₋₁₂ straight-chain alkyl, more preferably dodecyl.

In preferred embodiments, the active substance with skin anti-inflammatory function is selected from one or more of: retinoic acid, α-hydroxy acid, salicylic acid, azelaic acid, and α-bisabolol.

In the embodiments of this invention, the skin inflammatory disease is at least one disease selected from: superficial scars, hypertrophic scars, keloids, lupus vulgaris, leprosy ulcers, tinea favosa, kerion, cutaneous cryptococcosis, impetigo, acne, and dermatitis. In preferred embodiments, the skin inflammatory disease is at least one dermatitis selected from neurodermatitis, seborrheic dermatitis, and atopic dermatitis. Specifically, the neurodermatitis are selected from eczema, lichen, psoriasis, and pruritus, etc.; the seborrheic dermatitis are selected from psoriasis of the head and face, pityriasis rosea, tinea corporis, etc.; and the atopic dermatitis are selected from erythema, papules, papulovesicles, exudative crusts, lichenification, skin scratches, skin dryness, etc..

In the skin-care product composition and pharmaceutical composition of the invention, the molar ratio of the skincare active substance or the active substance with skin anti-inflammatory function to the sulfonated calixarene compound is 1:(0.8-5.0), preferably 1:(0.9-3.0), more preferably 1:(1.0-1.5).

Sulfonated calixarene compounds are a class of supramolecular macrocyclic compounds that can improve the solubility and stability of skincare ingredients, have a sustained-release effect on drugs, and reduce their irritation to normal cells.

The sulfonated calixarene compounds provided by this invention have the characteristics of accurate structure, fixed molecular weight, stable batch synthesis, easy derivatization, and unique cavity binding properties. They can be used to enhance the water solubility and stability of skincare active substances, protect the functions of each active ingredient from interfering with each other, and reduce the irritation of drugs to inflamed skin. Therefore, another purpose of this invention is to provide the use of the sulfonated calixarene compounds of formula (I) in the preparation of skincare products or drugs for treating skin inflammatory diseases.

### Figures Description

Figure 1 shows a schematic diagram of the host-guest inclusion complex formed between the sulfonated calixarene compound SC8A-12C of the invention and vitamin A or retinoic acid.
Figure 2a shows the UV titration curve of vitamin A with SC8A-12C (*λ* = 317 nm), with the arrow indicating the curves from top to bottom at 317 nm corresponding to the concentration of SC8A-12C from high to low; Figure 2b shows the binding constant fitting curve of Rh B with SC8A-12C, fitted by the 1:1 host-guest direct binding model (host: SC8A-12C, guest: vitamin A).
Figure 3 shows a comparison of the fluorescence intensity between the inclusion complex SC8A-12C-LCG and the same complex after the introduction of a large amount of Ca²⁺ and Mg²⁺.
Figure 4 shows the dissolution of phenylethyl resorcinol in water and the dissolution of phenylethyl resorcinol in water with the solubilization of SC8A-12C.
Figure 5 shows the UV absorption curves of vitamin A in water, vitamin A in water with methanol as a solvent aid, solutions of vitamin A solubilized by SC4A, SC8A and SC8A-12C.
Figure 6 shows the stabilizing effect of SC8A-12C on solubilized vitamin A.
Figure 7 shows the UV absorption curves of retinoic acid in water, retinoic acid in water with methanol as a solvent aid, solutions of retinoic acid solubilized by SC4A, SC8A, and SC8A-12C.
Figure 8 shows the release profile of vitamin A in SC8A-12C-vitamin A (inclusion complex).
Figure 9 shows the release profile of retinoic acid in SC8A-12C-retinoic acid (inclusion complex).
Figure 10 shows the UV absorption curves of the solutions in the receptor compartment after 24 hours of testing with an intelligent transdermal diffuser for the free retinoic acid group and the SC8A-12C-retinoic acid (inclusion complex) group.

### Detailed Description of the Invention

### Terminology

The term "alkyl" refers to an aliphatic hydrocarbon group, which may have branched or straight chain. Depending on the structure, an alkyl group can be a monoradical or a diradical (i.e., an alkylene group). In the invention, the alkyl group is preferably an alkyl having 1 to 8 carbon atoms, and more preferably a "lower alkyl" having 1 to 6 carbon atoms, and even more preferably an alkyl having 1 to 4 carbon atoms. Typical alkyl groups include, but are not limited to, methyl, ethyl, propyl, butyl, pentyl, hexyl, and the like. It should be understood that the "alkyl" mentioned herein includes all possible configurations and conformations of the alkyl group. For example, the "propyl" mentioned herein includes n-propyl and isopropyl, "butyl" includes n-butyl, isobutyl, and tert-butyl, "pentyl" includes n-pentyl, isopentyl, neopentyl, tert-pentyl, and pent-3-yl.

The term "alkoxy" refers to an -O-alkyl group, where the alkyl is as defined herein. Typical alkoxy groups include, but are not limited to, methoxy, ethoxy, propoxy, butoxy, pentyloxy, hexyloxy, and the like.

The term "cycloalkyl" refers to a monocyclic or polycyclic radical that contains only carbon and hydrogen. Cycloalkyl groups include groups having from 3 to 12 ring atoms. Depending on the structure, a cycloalkyl group can be a monoradical or a diradical (e.g., an cycloalkylene group). In the invention, the cycloalkyl group is preferably a cycloalkyl having 3 to 8 carbon atoms, and more preferably a "lower cycloalkyl" having 3 to 6 carbon atoms. Examples of cycloalkyl groups include, but are not limited to, cyclopropyl, cyclobutyl, cyclopentyl, cyclohexyl, cycloheptyl, cyclooctyl, cyclopentenyl, cyclohexenyl, cycloheptenyl, and adamantyl.

The term "aromatic" refers to a planar ring having a delocalized π-electron system containing 4n+2 π electrons, where n is an integer. Aromatic rings can be formed from five, six, seven, eight, nine, or more than nine atoms. Aromatics can be optionally substituted. The term "aromatic" includes both carbocyclic aryl (e.g., phenyl) and heterocyclic aryl (or "heteroaryl" or "heteroaromatic") groups (e.g., pyridine). The term includes monocyclic or fused-ring polycyclic (i.e., rings which share adjacent pairs of carbon atoms) groups.

As used herein, the term "aryl" refers to an aromatic ring wherein each of the atoms forming the ring is a carbon atom. Aryl rings can be formed from five, six, seven, eight, nine, or more than nine carbon atoms. Aryl groups can be optionally substituted. Examples of aryl groups include, but are not limited to phenyl, naphthalenyl, phenanthrenyl, anthracenyl, fluorenyl, and indenyl. Depending on the structure, an aryl group can be a monoradical or a diradical (i.e., an arylene group).

The term "aryloxy" refers to an -O-aryl group, wherein aryl is as defined herein.

The term "heteroaryl" refers to an aryl group that includes one or more ring heteroatoms selected from nitrogen, oxygen and sulfur. An N-containing "heteroaryl" moiety refers to an aromatic group in which at least one of the skeletal atoms of the ring is a nitrogen atom. Depending on the structure, the heteroaryl group may be a monoradical or a diradical (i.e., a heteroarylene group). Examples of heteroaryl groups include, but are not limited to pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, indazolyl, indolizinyl, phthalazinyl, pyridazinyl, isoindolyl, pteridinyl, purinyl, oxadiazolyl, thiadiazolyl, furyl, benzofuranyl, benzothiophenyl, benzothiazolyl, benzoxazolyl, quinazolinyl, naphthyridinyl, furopyridinyl, and the like.

As used herein, the term "heteroalkyl" refers to an alkyl radical, as defined herein, in which one or more skeletal chain atoms is a heteroatom, e.g., oxygen, nitrogen, sulfur, silicon, phosphorus or combinations thereof. The heteroatom(s) may be placed at any interior position of the heteroalkyl group or at the position at which the heteroalkyl group is attached to the remainder of the molecule.

As used herein, the term "heterocycloalkyl" or "heterocyclyl" refers to a non-aromatic ring wherein one or more atoms forming the ring is a heteroatom selected from nitrogen, oxygen and sulfur. Heterocycloalkyl rings can be formed by three, four, five, six, seven, eight, nine, or more than nine atoms. Heterocycloalkyl rings can be optionally substituted. Examples of heterocycloalkyls include, but are not limited to, lactams, lactones, cyclic imides, cyclic thioimides, cyclic carbamates, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, imidazolidine, pyrrolidone, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, and 1,3-oxathiolane. Depending on the structure, a heterocycloalkyl group can be a monoradical or a diradical (i.e., a heterocycloalkylene group).

The term "halo" or "halogen" refers to fluoro, chloro, bromo and iodo.

The terms "haloalkyl", "haloalkoxy" and "haloheteroalkyl" include alkyl, alkoxy or heteroalkyl structures in which at least one hydrogen is replaced with a halogen atom. In certain embodiments in which two or more hydrogen atoms are replaced with halogen atoms, the halogen atoms are the same or different as one another.

The term "amino" refers to a -NH₂ group.

The term "hydroxy" refers to a -OH group.

The term "cyano" refers to a -CN group.

The term "ester" refers to a chemical moiety having the formula -COOR, where R is selected from alkyl, cycloalkyl, aryl, heteroaryl (attached through a ring carbon), and heterocyclyl (attached through a ring carbon).

The term "amide" or "amido" refers to -NR-CO-R', where R and R' are each independently hydrogen or alkyl.

The term "aminoacyl" refers to a -CO-NH₂ group.

The term "alkylaminoacyl" refers to a -CO-NH-R group, where R is an alkyl group as defined herein.

The term "optionally" means that one or more events described hereinafter may or may not occur, and include both the event(s) that may occur and the event(s) that may not occur. The term "optionally substituted" or "substituted" refers to that the mentioned group may be substituted with one or more additional groups which are each independently selected from alkyl, cycloalkyl, aryl, heteroaryl, heterocyclyl, hydroxy, alkoxy, cyano, halo, amide, nitro, haloalkyl, amino, methylsulfonyl, alkylcarbonyl, alkoxy carbonyl, heteroarylalkyl, heterocycloalkylalkyl, aminoacyl, amino protecting group, etc., wherein, the amino protecting group is preferably selected from the group consisting of pivaloyl, tert-butoxycarbonyl, benzyloxycarbonyl, 9-fluorenylmethoxycarbonyl, benzyl, p-methoxybenzyl, allyloxycarbonyl, trifluoroacetyl, and the like.

Unless defined otherwise, all technical and scientific terms used herein have the same meaning as is commonly understood by one of skill in the art to which the claimed subject matter belongs.

Unless otherwise indicated, conventional methods of mass spectroscopy, NMR, HPLC, protein chemistry, biochemistry, recombinant DNA techniques and pharmacology, within the skill of the art are employed in the invention. Unless specific definitions are provided, the nomenclature employed in connection with, and the laboratory procedures and techniques of, analytical chemistry, synthetic organic chemistry, and medicinal and pharmaceutical chemistry described herein are those known in the art. The foregoing techniques and procedures can be generally performed of conventional methods well known in the art and as described in various general and more specific references that are cited and discussed throughout the specification.

### The sulfonated calixarene compounds of the invention and their uses in encapsulating skincare active substances

The invention provides a molecular container platform for encapsulating skincare active substances, which improves the water solubility and stability of the skincare active substances and protects the functions of the various ingredients from interfering with each other. The molecular container platform of the invention is a sulfonated calixarene compound of formula (I): 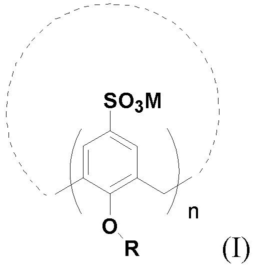 wherein,
n is an integer selected from 4 to 8,
M is independently selected from H, alkali metals, and alkaline earth metals,
R is independently selected from C₄₋₁₆ straight-chain alkyl.

In preferred embodiments, M is at least one metal selected from the group consisting of Na, K, Mg, and Ca, more preferably Na.

In other preferred embodiments, R is selected from C₈₋₁₂ straight-chain alkyl, more preferably dodecyl.

Particularly preferably, the sulfonated calixarene compound of the invention is selected from the following compounds:

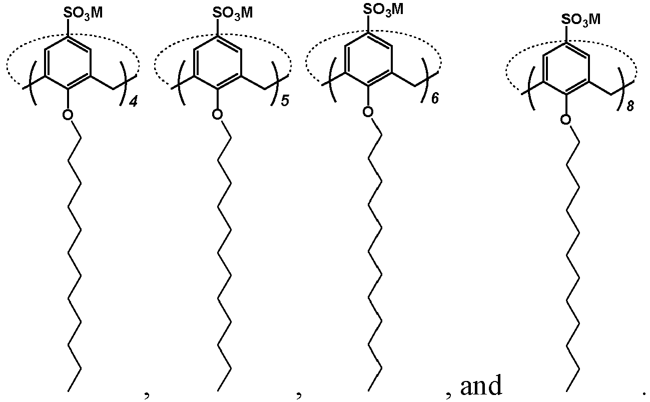

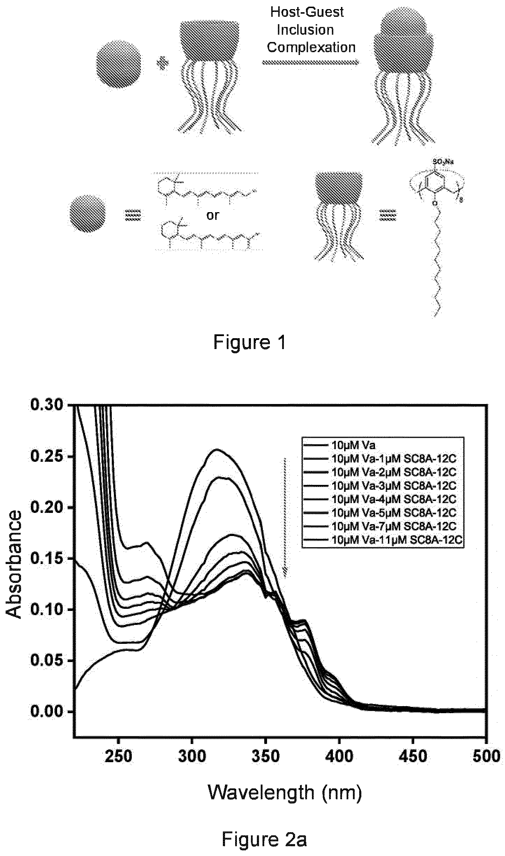

The sulfonated calixarene compound of the invention can form a host-guest inclusion complex with skincare active substances through non-covalent interactions such as hydrogen bonding, electrostatics, and hydrophobicity, with binding constants of 10³ or higher, more preferably 10⁴ or higher, and even more preferably 10⁵ or higher. As shown in Figure 1, the sulfonated calixarene compound of the invention can increase the solubility of fat-soluble active substances in water, and also enhance their stability. Using fat-soluble active substances in water makes the active substances more closely fit the skin, which helps enhance the permeability of the active substances. Therefore, the sulfonated calixarene compound of the invention helps reduce the amount of skincare molecules used and enhances efficacy. Moreover, the sulfonated calixarene compound of the invention has a sustained-release effect on skincare active substances, so that the skincare active substances are controllably released, reducing the irritation to normal cells.

Since the sulfonated calixarene compound of the invention can form a stable host-guest non-covalent binding structure with a variety of small-molecule skincare active substances, and reduce the irritation of skincare active substances on inflamed skin, it can be used as a molecular container platform to enhance the efficacy of skincare products in the skincare field.

### The novel supramolecular skin-care product composition and pharmaceutical composition of the invention

The invention also provides a skin-care product composition or a pharmaceutical composition for treating skin inflammatory diseases, comprising at least one skincare active substance and a sulfonated calixarene compound of formula (I). Optionally, the skin-care product composition or pharmaceutical composition of the invention may also include other carriers acceptable in the skincare industry and other functional ingredients or pharmaceutical ingredients.

In preferred embodiments, the skincare active substances used for solubilization, stabilization, and specific release using the sulfonated calixarene molecular container platform of the invention can be used to meet one or more of the following skincare needs: cleansing or makeup removal, whitening, anti-aging (such as anti-wrinkle, antioxidant), anti-inflammatory or anti-acne, moisturizing, and sun protection.

Preferably, the active substances with cleansing or makeup removal functions are selected from: polyols such as butylene glycol, polyethylene glycol, and dipropylene glycol, surfactants such as sodium dodecyl sulfate, sodium laureth sulfate, sodium acyl sulfate, decyl glucoside, cocamidopropyl betaine, and amino acids, and synthetic esters such as isopropyl myristate, isopropyl hexadecanoate, and triglyceride.

The active substances with whitening functions are selected from: vitamin C and derivatives thereof (such as sodium/magnesium ascorbyl phosphate, ascorbyl glucoside, ethyl ascorbic acid, and ascorbyl tetraisopalmitate), phenylethyl resorcinol, niacinamide, tranexamic acid, arbutin, ellagic acid, p-dihydroxybenzene, kojic acid, glutathione, salicylic acid, tretinoin, and hydroquinone.

The active substances with anti-aging functions are selected from: vitamin A, proanthocyanidins, vitamin E, resveratrol, peptides (such as hexapeptide, palmitoyl tripeptide-5), bifida ferment lysate, astaxanthin, epidermal growth factor, glycolic acid and lactic acid, ubiquinone, caffeine, glycolic acid, lactic acid, vitamin C and derivatives thereof.

The active substances with anti-inflammatory or anti-acne functions are selected from: retinoic acid, α-hydroxy acid, salicylic acid, azelaic acid, and α-bisabolol.

The active substances with moisturizing functions are selected from: ceramides, sphingolipids, phospholipids, cholesterol, lecithin, squalane, hyaluronic acid, chondroitin sulfate, natural moisturizing factor, sorbitol, mannitol, glucose, trehalose, glycerin, pentylene glycol, butylene glycol, and provitamin B5.

The active substances with sun protection functions are selected from: benzophenone-3, octyl methoxycinnamate, ensulizole, PEG-25 p-aminobenzoic acid, ethylhexyl triazone, 2-ethylhexyl 2-cyano-3,3-diphenylacrylate, pentyl Padimate-O, polyacrylamide methylbenzylidene camphor, glyceryl p-aminobenzoate, diethylamino hydroxybenzoyl hexyl benzoate, camphor benzalkonium methosulfate, benzophenone-5, homosalate, ethylhexyl methoxycinnamate, isoamyl methoxycinnamate, and disodium phenyl dibenzimidazole tetrakisulfonate.

In the skin-care product composition and pharmaceutical composition of the invention, the molar ratio of skincare active substance or active substance with skin anti-inflammatory function to the sulfonated calixarene compound is 1:(0.8-5.0), preferably 1:(0.9-3.0), more preferably 1:(1.0-1.5).

In use, a single skincare active substance or a combination of multiple skincare active substances can be used according to the situation. The sulfonated calixarene molecular container platform of the invention can be used in the following products: skincare products, such as skincare creams, lotions, oils, toners, talcum powders, bath products, eye skincare products, facial masks, and facial cleansers; cosmetics, such as foundations, pressed powders, rouges, body makeup, eyebrow pencils, eye shadows, eyelid products, eyelash products, eye makeup removers, lip balms, lip glosses, general color lipsticks, lip liners.

In the embodiments of the invention, when improving the skin quality of consumers according to the invention, the selected skincare products depend on many factors, such as the skin type of consumers, and then select appropriate skincare products, and follow the correct steps for skincare. The general skincare and makeup steps are as follows: 1. Clean the face, 2. Serum, 3. Toner, 4. Eye essence, 5. Facial essence, 6. Eye cream, 7. Lotion, 8. Face cream; Next is the makeup steps: 1. Apply lip balm first, 2. Sunscreen, 3. Base makeup, 4. Concealer, 5. Set makeup, 6. Draw eyebrows, 7. Eyebrow dye, 8. Eye shadow, 9. Eyeliner, 10. Eyelash curler, 11. False eyelashes, 12. Eyelash primer, 13. Mascara, 14. Contouring, 15. Highlight, 16. Nose shadow, 17. Draw lip line, 18. Lipstick, 19. Blush, 20. Set makeup again. It is worth noting that makeup removal, exfoliation, and facial masks are also indispensable. Those skilled in the art will understand that, although the above skincare suggestions are given, the specific operating steps and usage amounts can be appropriately adjusted according to the needs of the consumers themselves and in combination with the guidance of professionals.

In addition to providing a sustained-release effect on skincare active substances and reducing the irritation of active substances on inflammatory skin, the advantages of the sulfonated calixarene compound of the invention as a molecular container platform include its high solubility in aqueous solution and its ability to provide additional solubilization for poorly soluble skincare active substances. Therefore, when used as an active substance delivery platform, the use concentration of the active substance is not restricted by solubility limitations, offering greater flexibility in selecting the concentration of use. Additionally, it avoids the greasy feeling associated with lipid matrices, enhancing the user experience.

The solubility of skincare active substances in solution can be enhanced through various methods, including co-grinding the active substances with the sulfonated calixarene compound of the invention, and followed by dissolution, or by subjecting them to ultrasonic treatment, shaking, or refluxing together in the solution. Preferably, when the skin-care product composition and pharmaceutical composition of the invention are in solution form, the concentration of the sulfonated calixarene compound in solution is 0.5-5 mM.

### Preparation of sulfonated calixarene compounds

Compounds of formula (I) can be synthesized using standard synthetic techniques known to those skilled in the art or by combining methods known in the art with the those described herein. Additionally, the solvents, temperatures and other reaction conditions given herein can be varied according to the practices in the art. As further guidance, the following synthetic methods can also be utilized.

The reactions described can be used sequentially to provide the compounds described herein; or they can be used to synthesize fragments, and the fragments are subsequently added by the methods described herein and/or methods known in the art.

Compounds can be synthesized using methods similar to those described below by using appropriate alternative starting materials. The starting materials used to synthesize the compounds described herein can either be synthesized or obtained from commercial sources. The compounds described herein and other related compounds having different substituents can be synthesized using techniques and starting materials known to those skilled in the art. The general methods for preparing the compounds disclosed herein can be derived from known reactions in the art, and these reactions can be modified by reagents and conditions deemed appropriate by those skilled in the art to introduce various moieties in the molecules provided herein.

If necessary, the reaction products can be separated and purified using conventional techniques, including but not limited to filtration, distillation, crystallization, chromatography and the like. These products can be characterized using conventional methods, including physical constants and spectral data.

Taking the sulfonated calixarene compound in which R is dodecyl as an example, its synthesis route is as follows.

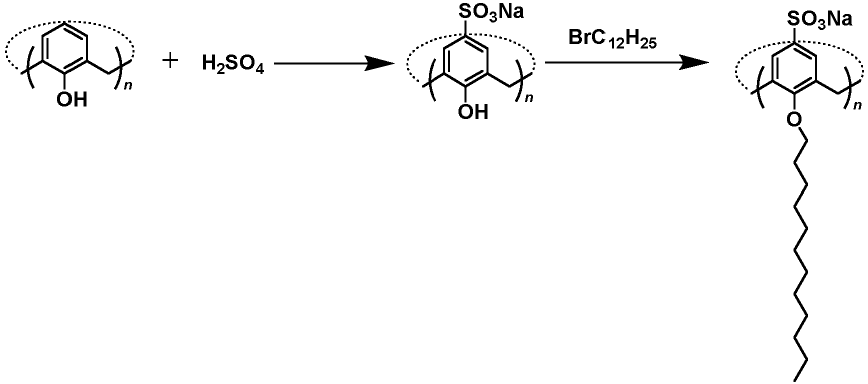

SC4A-12C: Calix[4]arene (calix[n]arene, where n is 4-8, synthesis process referenced in Org. Synth. 1990, 68, 238; J. Am. Chem. Soc. 1982, 104, 2652; J. Org. Chem. 1998, 63, 489) (2.00 g, 4.71 mmol) was added to a round-bottom flask. 12 mL of concentrated sulfuric acid was slowly added, and the reaction was carried out at 90°C for 4 hours. A small amount of the reaction solution was taken and added to water. If no insoluble material was observed, the heating was stopped, the reaction solution was cooled to room temperature. The reaction solution was then added dropwise to 400 mL of rapidly stirred ethyl ether, stirred overnight, and subjected to suction filtration, and an off-white solid was obtained. The solid was dissolved in a small amount of water, and sodium hydroxide (0.77 g, 19.25 mmol) was added. A large amount of ethanol was then added to induce recrystallization. Sulfonated calix[4]arene (SC4A) was obtained via suction filtration. SC4A (3.00 g, 3.60 mmol) and sodium hydroxide (2.35 g, 58.8 mmol) were dissolved in 15 mL of water and stirred for 3 hours. Then, dodecyl bromide (15.40 g, 61.8 mmol) dissolved in 60 mL of DMSO was added, and the mixture was reacted at 100°C for 5 days. After cooling to room temperature, a large amount of methanol was added under stirring, and the mixture was placed in a refrigerator to stand in the freezer. The mixture was subjected to suction filtration, the solid was dissolved in water, and insoluble materials were filtered out. A large amount of ethanol was added under stirring, and the mixture was placed in the refrigerator to stand in the freezer. After suction filtration and drying, 4.99 g of the product sulfonated calix[4]arene compound (SC4A-12C) was obtained, with a yield of 92%. 1H NMR (400 MHz, DMSO-d6) δ 7.08 (s, 8H, ArH), 4.32 (d, 4H, Ar-CH2-Ar), 3.86 (t, 8H, Ar-O-CH2), 3.22 (d, 4H, Ar-CH2-Ar), 1.92 (m, 8H, -O-CH2-CH2), 1.37-1.24 (m, 72H, alkyl chain), 0.85 (t, 12H, CH3).

SC6A-12C: Calix[6]arene (2.00 g, 3.14 mmol) was added to a round-bottom flask. 12 mL of concentrated sulfuric acid was slowly added, and the reaction was carried out at 90°C for 4 hours. A small amount of the reaction solution was taken and added to water. If no insoluble material was observed, the heating was stopped, the reaction solution was cooled to room temperature. The reaction solution was then added dropwise to 400 mL of rapidly stirred ethyl ether, stirred overnight, and subjected to suction filtration, and an off-white solid was obtained. The solid was dissolved in a small amount of water, and sodium hydroxide (0.77 g, 19.25 mmol) was added. A large amount of ethanol was then added to induce recrystallization. Sulfonated calix[6]arene (SC6A) was obtained via suction filtration. SC6A (3.00 g, 2.40 mmol) and sodium hydroxide (2.35 g, 58.8 mmol) were dissolved in 15 mL of water and stirred for 3 hours. Then, dodecyl bromide (15.40 g, 61.8 mmol) dissolved in 60 mL of DMSO was added, and the mixture was reacted at 100°C for 5 days. After cooling to room temperature, a large amount of methanol was added under stirring, and the mixture was placed in a refrigerator to stand in the freezer. The mixture was subjected to suction filtration, the solid was dissolved in water, and insoluble materials were filtered out. A large amount of ethanol was added under stirring, and the mixture was placed in the refrigerator to stand in the freezer. After suction filtration and drying, 5.20 g of the product sulfonated calix[6]arene compound (SC6A-12C) was obtained, with a yield of 96%. 1H NMR (400 MHz, DMSO-d6) δ 7.37 (s, 12H, ArH), 4.54 (d, 6H, Ar-CH2-Ar), 3.67 (t, 12H, Ar-O-CH2), 3.43 (d, 6H, Ar-CH2-Ar), 1.82 (m, 12H, -O-CH2-CH2), 1.31-1.22 (m, 108H, alkyl chain), 0.85 (t, 18H, CH3)_{∘}

SC8A-12C: Calix[8]arene (2.00 g, 2.36 mmol) was added to a round-bottom flask. 12 mL of concentrated sulfuric acid was slowly added, and the reaction was carried out at 90°C for 4 hours. A small amount of the reaction solution was taken and added to water. If no insoluble material was observed, the heating was stopped, the reaction solution was cooled to room temperature. The reaction solution was then added dropwise to 400 mL of rapidly stirred ethyl ether, stirred overnight, and subjected to suction filtrations, and an off-white solid was obtained. The solid was dissolved in a small amount of water, and sodium hydroxide (0.77 g, 19.25 mmol) was added. A large amount of ethanol was then added to induce recrystallization. Sulfonated calix[8]arene (SC8A) was obtained via suction filtration. SC8A (3.00 g, 1.80 mmol) and sodium hydroxide (2.35 g, 58.8 mmol) were dissolved in 15 mL of water and stirred for 3 hours. Then, dodecyl bromide (15.40 g, 61.8 mmol) dissolved in 60 mL of DMSO was added, and the mixture was reacted at 100°C for 5 days. After cooling to room temperature, a large amount of methanol was added under stirring, and the mixture was placed in a refrigerator to stand in the freezer. The mixture was subjected to suction filtration, the solid was dissolved in water, and insoluble materials were filtered out. A large amount of ethanol was added under stirring, and the mixture was placed in the refrigerator to stand in the freezer. After suction filtration and drying, 4.89 g of the product sulfonated calix[8]arene compound (SC8A-12C) was obtained, with a yield of 90%. 1H NMR (400 MHz, DMSO-d6) δ 7.27 (s, 16H, ArH), 4.32 (d, 8H, Ar-CH2-Ar), 3.66-3.56 (m, 24H, Ar-CH2-Ar, Ar-O-CH2), 1.63 (m, 16H, -O-CH2-CH2), 1.37-1.24 (m, 144H, alkyl chain), 0.84 (t, 24H, CH3)_{∘}

### Test Example

### Example 1: Determination of Binding Constants Between Sulfonated Calixarene Compounds and Skincare Active Substances

### Test Method: UV titration.

Test Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

Reagents: Vitamin A (Va) was purchased from Shanghai Macklin Biochemical Technology Co., Ltd, retinoic acid was purchased from Heowns Biochem Technologies, LLC, Tianjin, and other skincare active substances were commercially available products.

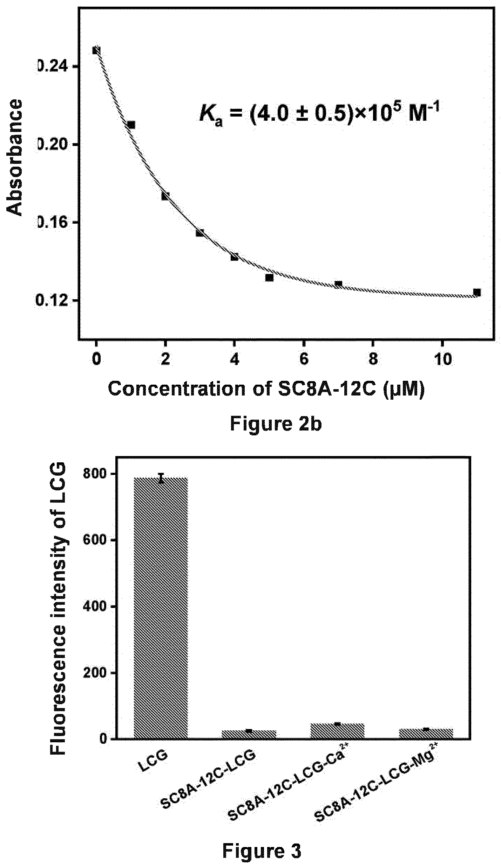

The UV titration experiments of SC8A-12C and active substances were carried out at room temperature (25°C). To titrate the binding constant of SC8A-12C with Va, SC8A-12C stock solution and Va stock solution were first prepared. SC8A-12C was dissolved in deionized water to a preparation concentration of 4 mM. Va was dissolved in methanol to a concentration of 2 mM. During the test, the Va stock solution was added to a cuvette and adjusted to a volume of 3 mL with deionized water, resulting in a concentration of 10 µM. Subsequently, the SC8A-12C stock solution was added to the cuvette in predetermined volumes in multiple aliquots, so that the final concentration of SC8A-12C increased successively (the final concentrations of SC8A-12C are shown in Figure 2a). The change in UV absorption intensity was recorded after each addition of the SC8A-12C stock solution. The UV titration data were fitted using a 1:1 host-guest direct binding model (host: SC8A-12C, guest: Va) at a wavelength of 317 nm. The binding constant *K_{α}* for the host-guest inclusion was determined. The results are shown in Figures 2a and 2b. The binding constant *K_{α}* of SC8A-12C for Va was (4.0 ± 0.5) ×10⁵ M⁻¹.

The binding constant of SC8A-12C with retinoic acid was determined using the same method. Specifically, the UV titration experiments of SC8A-12C with retinoic acid were carried out at room temperature (25°C). SC8A-12C stock solution and retinoic acid stock solution were first prepared. SC8A-12C was dissolved in deionized water to a preparation concentration of 4 mM, retinoic acid was dissolved in methanol to a preparation concentration of 2 mM. During the test, the retinoic acid stock solution was added to a cuvette and adjusted to a volume of 3 mL with deionized water, resulting in a concentration of 15 µM. Subsequently, the SC8A-12C stock solution was added to the cuvette in predetermined volumes in multiple aliquots, so that the final concentration of SC8A-12C increased successively. The change in UV absorption intensity was recorded after each addition of the SC8A-12C stock solution. The binding constant *K_{α}* of SC8A-12C for retinoic acid was (1.0 ± 0.3) × 10⁵ M⁻¹.

The binding constants of SC8A-12C with various active substances were determined using the same method. Specifically, the UV titration experiments of SC8A-12C with active substances were carried out at room temperature (25°C). Stock solutions of SC8A-12C and active substances were first prepared, separately. SC8A-12C was dissolved in deionized water to a preparation concentration of 2 mM, and the active substance was dissolved in methanol or water to a preparation concentration of 2 mM. During the test, the stock solution of the active substance was added to a cuvette and adjusted to a volume of 3 mL with deionized water, resulting in a concentration of 15 µM. Subsequently, the SC8A-12C stock solution was added to the cuvette in predetermined volumes in multiple aliquots, so that the final concentration of SC8A-12C increased successively. The change in UV absorption intensity was recorded after each addition of the SC8A-12C stock solution. The UV titration data were fitted using a 1:1 host-guest direct binding model to determine the binding constant *K_{α}* for the host-guest inclusion. The test results of the binding constants of various active substances with SC8A-12C are shown in Table 1.

**Table 1. Binding constants (K_{α}) of SC8A-12C with skincare active substances**

| Types of active substances | Drug Names | Binding constants *K*ₐ/M⁻¹ |
|---|---|---|
| active substancess with cleansing or makeup removal functions | cocamidopropyl betaine | 0.8±0.1×10⁴ |
| | decyl glucoside | 3.3±0.5×10⁴ |
| | isopropyl hexadecanoate | 2.2±0.4×10³ |
| active substances with anti-aging functions | vitamin A | 4.4±0.5×10⁵ |
| | proanthocyanidins | 2.6±0.3×10³ |
| | vitamin E | 2.6±0.4×10³ |
| | hexapeptide | 5.2±0.8×10³ |
| | palmitoyl tripeptide-5 | 6.3±0.6×10³ |
| | caffeine | 3.2±0.8×10³ |
| active substances with whitening functions | ascorbyl glucoside | 5.2±0.3×10³ |
| | arbutin | 4.3±0.5×10³ |
| | phenylethyl resorcinol | 4.5±0.5×10⁴ |
| active substances with anti-inflammatory or anti-acne functions | retinoic acid | 1.0±0.3×10⁵ |
| | α-bisabolol | 2.8±0.6×10⁴ |
| | azelaic acid | 5.2±0.1×10³ |
| active substances with moisturizing functions | cholesterol | 2.3±0.5×10⁵ |
| | sphingolipids | 2.5±0.5×10³ |
| | ceramides | 6.5±0.2×10³ |
| | sorbitol | 5.3±0.4×10³ |
| active substances with sun protection functions | diethylamino hydroxybenzoyl hexyl benzoate | 3.8±0.3×10³ |
| | glyceryl p-aminobenzoate | 2.9±0.2×10³ |

The higher binding constant of the host-guest complex indicates a stronger inclusion ability of SC8A-12C with the active substances, and higher stability of the formed inclusion complex, making the skincare active substance less likely to leak.

As shown in the experimental results, the sulfonated calixarene compounds of the invention exhibited strong inclusion strength with skincare active substances, with binding constants of 10³ or higher, preferably 10⁴ or higher, and more preferably 10⁵ or higher. Therefore, the sulfonated calixarene compounds of the invention can form stable non-covalent host-guest binding with active substances.

### Example 2: Experiments on the Interference of Ca²⁺ and Mg²⁺ on Host-Guest Inclusion

### Test Method: Fluorescence Spectroscopy.

Test Equipment: Quartz cuvettes were used as sample cells with an optical path length of 10 mm. The instrument type was Varian Cary Eclipse, equipped with a Cary Single-cuvette Peltier cuvette temperature control unit.

First, a stock solution of Lucigenin (LCG) (purchased from Beijing Oaknas Biotechnology Co., Ltd.) was prepared by dissolving it in water to a preparation concentration of 1 mM. Then, a stock solution of SC8A-12C was prepared by dissolving it in deionized water to a preparation concentration of 1 mM. The LCG stock solution was added to a cuvette and adjusted to a volume of 3 mL with deionized water, resulting in a concentration of 10 µM. The excitation wavelength was set at 365 nm, and the emission wavelength at 505 nm. The fluorescence intensity of 10 µM LCG at 505 nm was first measured. Subsequently, SC8A-12C stock solution was added to achieve a concentration of 10 µM of SC8A-12C, and the fluorescence intensity of the 10 µM SC8A-12C-LCG at 505 nm was measured. Next, a solution containing 10 µM SC8A-12C-LCG and 2.5 mM Ca²⁺ and a solution containing 10 µM SC8A-12C-LCG and 0.8 mM Mg²⁺ were prepared, and the fluorescence intensity of these two solutions at 505 nm was measured. The test results are shown in Figure 3.

The results indicate that the addition of large amounts of Ca²⁺ and Mg²⁺ to the inclusion complex SC8A-12C-LCG has no effect on the binding. This suggests that even if the metal ions on the head group of SC8A-12C are replaced by Ca²⁺, Mg²⁺, or other ions, calixarene can still encapsulate guest molecules through host-guest interactions.

### Example 3: Experiments on the Solubilization of Phenylethyl Resorcinol by Sulfonated Calixarene

### Test Method: UV Absorption Spectroscopy.

Test Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

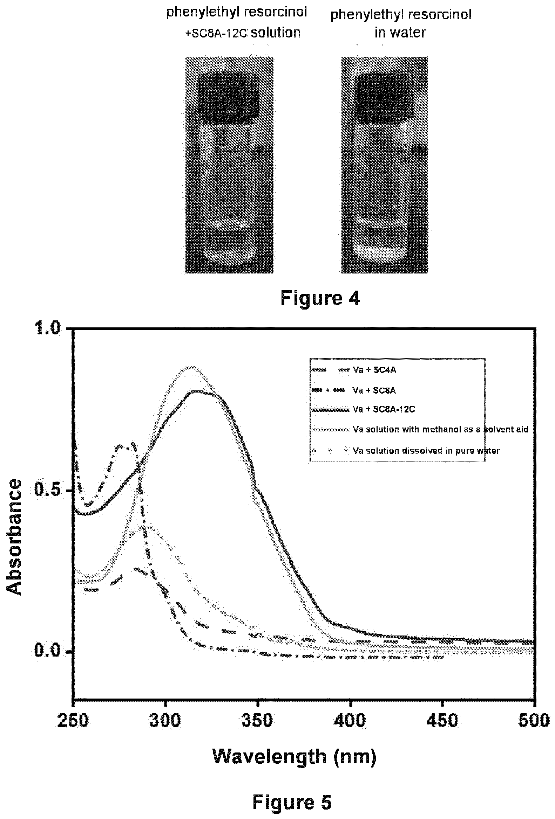

Standard solutions of phenylethyl resorcinol with concentrations of 100, 80, 60, 40, and 20 µg/mL were prepared in deionized water. The UV absorption intensities were measured, and a standard working curve was prepared. 10 mg of phenylethyl resorcinol was added to 1 mL deionized water and 1 mL of 2 mM SC8A-12C solution, separately. The mixtures were shaken overnight and then centrifuged to collect the supernatant for UV absorption curve detection. The supernatant of the phenylethyl resorcinol aqueous solution was diluted to an appropriate concentration (to match the standard working curve) for UV absorption intensity measurement (Figure 4).

Figure 4 shows that 1 mL of a 2 mM SC8A-12C solution completely dissolved 10 mg of phenylethyl resorcinol, yielding a clear and transparent solution; whereas 1 mL of deionized water could not dissolve 10 mg of phenylethyl resorcinol completely, leaving white precipitates of phenylethyl resorcinol at the bottom of the container.

According to the standard working curve, the solubility of phenylethyl resorcinol in water was calculated to be 4.9 mg/mL; whereas the solubility of phenylethyl resorcinol in water, enhanced by the sulfonated calixarene of the invention, was at least above 10 mg/mL (completely dissolved).

### Example 4: Experiments on the Solubilization of Vitamin A by Sulfonated Calixarene

### Testing Method: UV Absorption Spectroscopy.

Testing Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

In 3 mL of deionized water, 5 mg (an excess amount) of Vitamin A was added, and the mixture was shaken overnight. The mixture was then centrifuged to collect the supernatant, and the UV Absorption Curve of the supernatant was measured (Figure 5).

Solutions of SC4A, SC8A, and SC8A-12C were each prepared at a concentration of 500 µM in deionized water. Excess Va was added to each of these three solutions at a concentration of 5 mg/mL and shaken on a shaker to enhance the solubility of Va. After 24 hours, each group of solution was diluted 40-fold for UV absorption wavelength measurement. Meanwhile, Va was dissolved in methanol solution to prepare a 4 mM Va methanol stock solution. The Va methanol stock solution was then diluted in water to prepare a 5 µM Va standard solution with methanol as a solvent aid, which was used for UV absorption wavelength measurement. The results are shown in Figure 5.

Figure 5 shows that the supernatant, in which vitamin A dissolved only with deionized water, did not exhibit the characteristic absorption peak of Va (with a maximum absorption wavelength of 317 nm). Instead, the UV absorption curve of the supernatant had a maximum absorption peak at 290 nm, which is likely due to dissolved impurities. This result indicates that Va is almost insoluble in water. In contrast, the freshly prepared Va standard solution (with methanol as a solvent aid) exhibited a characteristic absorption peak of Va at 320 nm, indicating that Va is soluble in water with the aid of methanol.

Compared with the Va standard solution with methanol as a solvent aid (maximum absorption wavelength at 317 nm), the characteristic absorption peak of Va was not observed in the solutions of SC4A and SC8A groups, indicating that they failed to solubilize Va. The UV absorption curves of these two groups of solutions had maximum absorption peaks around 280 nm, which are the inherent absorption peaks of the calixarenes themselves. SC8A-12C exhibited a good solubilization effect on Va.

Following, solutions of SC8A-12C with concentrations of 250, 500, 1000, 2000, and 4000 µM were prepared in deionized water, separately. An excess amount of Va was added to each of these solutions at 5 mg/mL, and the solutions were agitated on a shaking table to enhance the solubility of Va. After 24 hours, each group of solutions was diluted by an appropriate multiple (to fit the standard working curve described below) and used for the measurement of UV absorbance intensity.

A standard working curve for Va was prepared. A methanol solution containing 4 mM of Va was prepared. A certain volume of the Va methanol solution was diluted into a solution containing 100 µM SC8A-12C, resulting in concentrations of Va in the diluted solutions of 40, 30, 20, 10, 5, and 2.5 µM, separately. The addition of 100 µM SC8A-12C was to eliminate the effect of SC8A-12C on the UV absorption of Va. Subsequently, the UV absorbance intensities of these six groups of solutions with different concentrations of Va were measured, and a standard working curve was prepared.

Based on the standard working curve, the amount of Va solubilized in the SC8A-12C solutions of different concentrations was calculated from the UV absorbance intensities. The results are shown in Table 2.

**Table 2. The Solubilization Capacity of SC8A-12C at Different Concentrations on Vitamin A**

| | | | | | | |
|---|---|---|---|---|---|---|
| [SC8A-12C]/µM | 0 | 250 | 500 | 1000 | 2000 | 4000 |
| [dissolved Va]/µM | 0* | 552 | 890 | 1616 | 1846 | 3375 |
| *Not detected under UV | | | | | | |

The results show that the sulfonated calixarene compound SC8A-12C of the invention has a significant solubilizing effect on Va, which is difficult to dissolve in water. Therefore, it can solubilize insoluble skincare molecules, enhancing the overall efficiency of active substances.

### Example 5: Experiments on the Stabilization of Skincare Active Substances by Sulfonated Calixarene

### Testing Method: UV-Visible Spectrophotometry.

Testing Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

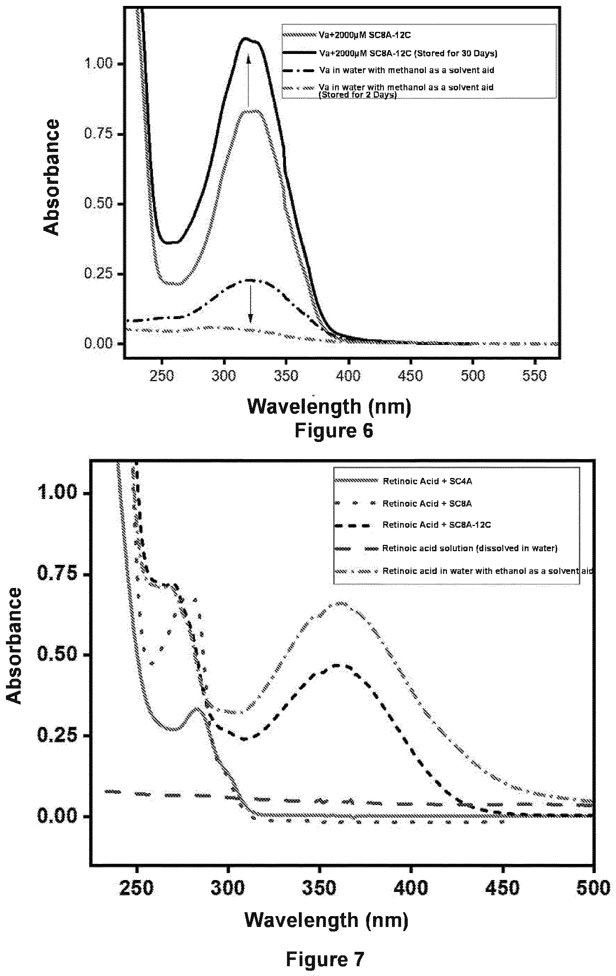

Experiments on the stabilization of Va by SC8A-12C were conducted as follows: A solution of SC8A-12C with a concentration of 2000 µM was prepared, to which an excess of Va was added at 5 mg/mL. After shaking to dissolve Va for 12 hours, the supernatant of the SC8A-12C solution was collected, diluted (to achieve a concentration of 15 µM SC8A-12C in the diluted supernatant), and then subjected to UV absorbance detection. After allowing the 2000 µM SC8A-12C solution to stand at room temperature for 30 days, the supernatant was collected, diluted to a solution with a SC8A-12C concentration of 15 µM, and then subjected to UV detection. The results are shown in Figure 6.

Va was dissolved in methanol to prepare a Va methanol solution with a concentration of 4 mM, which was then kept in the dark for two days. The UV absorptions of both the freshly prepared and the two-day stored Va methanol solutions were tested. During the testing, the Va methanol solution was first added to a cuvette, and deionized water was used to adjust the volume to 3 mL, achieving a final concentration of Va at 10 µM. The UV absorption curve was detected (shown in Figure 6).

From Figure 6, it can be seen that the freshly prepared Va aqueous solution (with methanol as a solvent aid) exhibited a characteristic absorption peak at 317 nm. However, after being stored for two days, the UV absorption of the Va aqueous solution (with methanol as a solvent aid) had significantly decreased, indicating that most of the free Va in the water had degraded, suggesting poor stability of Va in water. After the SC8A-12C solution was stored for 30 days, the amount of solubilized Va actually increased. This is because there was an excess of elemental Va in the solution, and over time, more Va dissolved. Additionally, SC8A-12C has a good stabilizing effect on Va, which allows the solubilized Va concentration to increase continuously over time rather than decrease.

### Example 6: Experiments on the Solubilization of Retinoic acid by Sulfonated Calixarene

### Testing Method: UV Absorption Spectroscopy.

Testing Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

Solutions of SC4A, SC8A, and SC8A-12C were each prepared at a concentration of 2000 µM in deionized water. Excess retinoic acid was added to each of the three groups of solutions at a concentration of 5 mg/mL, followed by ultrasonic treatment to enhance the solubility of retinoic acid. After 20 mins, each group of solution was diluted 10-fold for UV absorption wavelength measurement. At the same time, 5 mg (an excess amount) of retinoic acid was added to 3 mL of deionized water, the mixture was shaken overnight, centrifuged to obtain the supernatant. The UV absorption curve of the supernatant was measured (Figure 7). Retinoic acid was dissolved in methanol solution to prepare a stock solution of 4 mM retinoic acid in methanol. After 20 mins, the stock solution of retinoic acid in methanol was diluted in water to prepare a retinoic acid standard solution with an appropriate concentration, for UV absorption wavelength detection. The results are shown in Figure 7.

Figure 7 shows that the supernatant, in which retinoic acid dissolved only with deionized water, did not exhibit the absorption peak of retinoic acid (with a maximum absorption wavelength of 360 nm), demonstrating that retinoic acid is hardly soluble in water. The retinoic acid standard solution (with methanol as a solvent aid) exhibited a characteristic absorption peak of retinoic acid at 360 nm, demonstrating that retinoic acid can be dissolved in water with the aid of methanol. In contrast to the retinoic acid standard solution, neither the SC4A nor the SC8A group exhibited a characteristic absorption peak of retinoic acid, indicating that they cannot solubilize retinoic acid effectively, while SC8A-12C demonstrated a better solubilizing effect on retinoic acid.

Following, solutions of SC8A-12C with concentrations of 250, 500, 1000, 2000, and 4000 µM were prepared in deionized water, separately. An excess amount of retinoic acid was added to the above solutions at 5 mg/mL, followed by ultrasonic treatment to enhance the solubility of retinoic acid. After 20 mins, each group of solutions was diluted by an appropriate multiple (to fit the standard working curve described below) and used for the measurement of UV absorbance intensity.

A standard working curve for retinoic acid was prepared. A methanol solution containing 4 mM of retinoic acid was prepared. A certain volume of the retinoic acid in methanol was diluted into a solution containing 100 µM SC8A-12C, resulting in concentrations of retinoic acid of 25, 20, 15, 10, 5, 2.5 µM, separately. The addition of 100 µM SC8A-12C was to eliminate the effect of SC8A-12C on the UV absorption of retinoic acid. Subsequently, the UV absorbance intensities of these six groups of solutions with different concentrations of retinoic acid were measured, and a standard working curve was prepared.

Based on the standard working curve, the amounts of retinoic acid solubilized in the SC8A-12C solutions of different concentrations were calculated, as shown in Table 3.

**Table 3. The Solubilization Capacity of SC8A-12C at Different Concentrations on Retinoic acid**

| | | | | | | |
|---|---|---|---|---|---|---|
| [SC8A-12C]/µM | 0 | 250 | 500 | 1000 | 1500 | 2000 |
| [dissolved retinoic acid] /µM | 0* | 35 | 84 | 160 | 126 | 305 |
| * Not detected under UV | | | | | | |

The results indicate that the sulfonated calixarene compounds of the invention exhibit a significant solubilizing effect on retinoic acid, which is poorly soluble in water. The poorly soluble skincare active substances can be solubilized so that they can be used in the aqueous phases, thereby improving the water solubility of the skincare active substances.

Example 7: Release Profile of Va from the Inclusion Complex SC8A-12C-Va

### Testing Method: UV absorption spectroscopy.

Testing Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

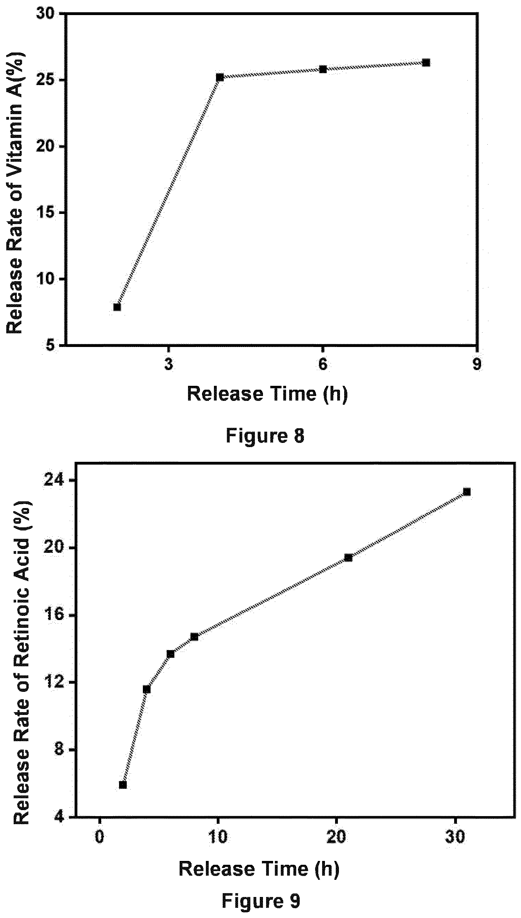

700 µL of SC8A-12C-Va solution with a Va concentration of 4 mM was placed in a dialysis bag with a molecular weight cutoff of 500 kDa. After tying both ends of the dialysis bag, it was put into a centrifuge tube containing 30 mL of deionized water. The centrifuge tube was placed in a shaker, and the solution in the centrifuge tube was taken for UV absorption detection after 2, 4, 6, and 8 hours. After each detection, the solution was recovered into the centrifuge tube. Finally, according to the standard working curve of Va obtained in Example 4, the content of Va released in the centrifuge tube was calculated, as shown in Figure 8.

The results show that after the inclusion of Va by SC8A-12C, a sustained release of Va is achieved, which is beneficial for reducing the irritation of Va and extending its effective duration.

Example 8: Release Profile of Retinoic acid from the Inclusion Complex SC8A-12C- Retinoic acid

### Testing Method: UV absorption spectroscopy.

Testing Equipment: A Japan Shimadzu UV-3600 UV-Visible Spectrophotometer, equipped with a temperature control module (model: PTC-348WI), was used. The samples were tested using Shimadzu's quartz cuvettes with an optical path length of 10 mm.

700 µL of SC8A-12C-Retinoic acid solution with a retinoic acid concentration of 664 µM was placed in a dialysis bag with a molecular weight cutoff of 500 kDa. After tying both ends of the dialysis bag, it was put into a centrifuge tube containing 15 mL of deionized water. The centrifuge tube was placed in a shaker, and the solution in the centrifuge tube was taken for UV absorption detection after 2, 4, 6, 8, 21, and 31 hours. After each detection, the solution was recovered into the centrifuge tube. Finally, according to the standard working curve of retinoic acid obtained in Example 6, the content of retinoic acid released in the centrifuge tube was calculated, as shown in Figure 9.

The results show that after the inclusion of retinoic acid by SC8A-12C, a sustained release of retinoic acid is achieved, which is beneficial for reducing the irritation of retinoic acid and extending its effective duration.

### Example 9: Experiment on the Promotion of Transdermal Permeability of Skincare Active Substance (Va) by Sulfonated Calixarene

### Testing Method: Skin Permeability Test

Testing Equipment: TP-6 Intelligent Transdermal Diffuser (Tianjin Jingtuo Instrument Technology Co., Ltd.)

The experiment to assess the promotion of transdermal permeability of Va by SC8A-12C was conducted as follows: Fresh pig skin obtained immediately post-slaughter was de-haired, and the intact skin was carefully excised. Undamaged sections were cut into uniform thickness pieces approximately 2 cm × 2 cm in size, wrapped in plastic wrap, and stored at -20°C for later use.

0.8 mg of Va and 200 mg of skincare cream (ointment base) were weighed and added to a mortar. The mixture was thoroughly ground to dissolve Va in the skincare cream, resulting in a Va concentration of 0.4% (w/w). Subsequently, 0.8 mg of Va was weighed and dispersed in 200 µL of water by thoroughly shaking to prepare a suspension of Va (water base), with a Va concentration of 0.4% (w/w). Next, 8.41 mg of SC8A-12C and 183.6 µL of water were weighed into a centrifuge tube and dissolved by heating. After SC8A-12C was dissolved, 0.8 mg of Va was added to prepare an inclusion complex. The concentration of Va in the inclusion complex was also 0.4% (w/w).

The skin was placed between the diffusion and receptor compartments after thawing. The receptor compartment was filled with the receptor solution (deionized water), and all air bubbles were expelled. The receptor compartment was maintained at 37°C in a circulating water bath throughout the experiment. The transdermal diffuser was activated, with a magnetic stirring speed set at 300 r·min⁻¹. After 55 hours, the solutions from the receptor compartments of each group were collected for UV detection.

The detection results showed that no Va was detected in the receptor compartment of the ointment base group, indicating that Va dissolved in the ointment base hardly penetrated through the pig skin. Va dissolved in water base existed in water as a large amount of undissolved Va, as oily material, due to its poor solubility, so its permeability was not good enough. However, compared with the group which Va dissolved in the ointment base, the water base was more conducive to the permeation of Va. The Va content detected in the receptor compartment of the SC8A-12C-Va group was 3.5 times that of the water base group, indicating that Va solubilized by SC8A-12C exhibited better permeation.

### Example 10: Experiments on the Promotion of Transdermal Permeability of Skincare Active Substance (Retinoic acid) by Sulfonated Calixarene

### Testing Method: Skin Permeability Test

Testing Equipment: TP-6 Intelligent Transdermal Diffuser (Tianjin Jingtuo Instrument Technology Co., Ltd.)

The experiment to assess the promotion of transdermal permeability of retinoic acid by SC8A-12C was conducted as follows: Fresh pig skin obtained immediately post-slaughter was de-haired, and the intact skin was carefully excised. Undamaged sections were cut into uniform thickness pieces, approximately 2 cm × 2 cm in size, wrapped in plastic wrap, and stored at -20°C for later use.

An excess amount of retinoic acid (5 mg/mL) was added to a 4 mM SC8A-12C solution. After subjecting to ultrasonic treatment for 30 min, the mixture was centrifuged to obtain the supernatant, which was SC8A-12C-retinoic acid inclusion complex group. The concentration of retinoic acid in the inclusion complex was determined by UV detection, and subsequently diluted to 0.1% (w/w). Additionally, 3 mg of retinoic acid was weighed and mixed with 297 mg of cream in a mortar. The mixture was thoroughly ground to dissolve retinoic acid in the cream, resulting in a retinoic acid concentration of 1% (w/w).

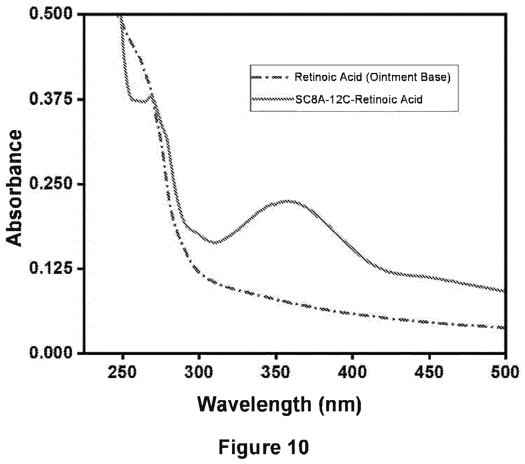

The skin was placed between the diffusion and receptor compartments after thawing. The receptor compartment was filled with the receptor solution (deionized water), and all air bubbles were expelled. The receptor compartment was maintained at 37°C in a circulating water bath throughout the experiment. The transdermal diffuser was activated, with a magnetic stirring speed set at 300 r·min⁻¹. After 24 hours, the solutions from the receptor compartments of each group were collected for UV detection. The results are shown in Figure 10.

As shown in Figure 10, no absorption peak of retinoic acid was detected in the receptor compartment of the ointment base group, indicating that retinoic acid dissolved in the ointment base hardly penetrated through the pig skin. In contrast, a distinct absorption peak of retinoic acid was detected in the receptor compartment of the SC8A-12C-retinoic acid inclusion complex group, demonstrating that the solubilization of retinoic acid by SC8A-12C facilitates its permeation.

Overall, the sulfonated calixarene compound of the invention enhances the water solubility and stability of active substances and achieve the sustained release of the active substances. When used in aqueous phase, the sulfonated calixarene compound improves the permeability of active substances and reduces their effective concentration. Furthermore, for irritating skincare active substances, the strong inclusion complexation and sustained release can mitigate the irritation of the skincare active substances to normal skin. Additionally, the enhanced stability of the active substances allows for reduced usage levels, which further contributes to mitigating irritation.

### Industrial Applicability

The invention provides a skin-care product composition and a pharmaceutical composition that utilize a sulfonated calixarene supramolecular compound as an auxiliary material, which can encapsulate a variety of skincare active substances to produce corresponding multifunctional skincare products, which exhibits solubilization, stabilization, enhanced permeability, and reduced irritation for a variety of skincare active substances, making it suitable for industrial application.

## Claims

1. A skin-care product composition, comprising at least one skincare active substance and a sulfonated calixarene compound of formula (I): 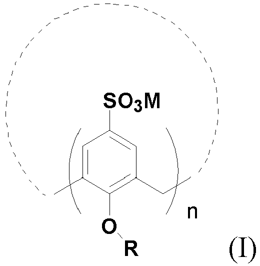 wherein,
n is an integer selected from 4 to 8,
M is independently selected from H, alkali metals, and alkaline earth metals,
R is independently selected from C₈₋₁₂ straight-chain alkyl,
the at least one skincare active substance is selected from active substances that have at least one of the following functions: cleansing or makeup removal, whitening, anti-aging, anti-inflammatory or anti-acne, moisturizing, and sun protection.

2. The skin-care product composition according to claim 1, wherein M is at least one metal selected from the group consisting of Na, K, Mg, and Ca;
and/or wherein R is dodecyl.

3. The skin-care product composition according to claim 1 or 2, wherein the sulfonated calixarene compound of formula (I) is selected from:

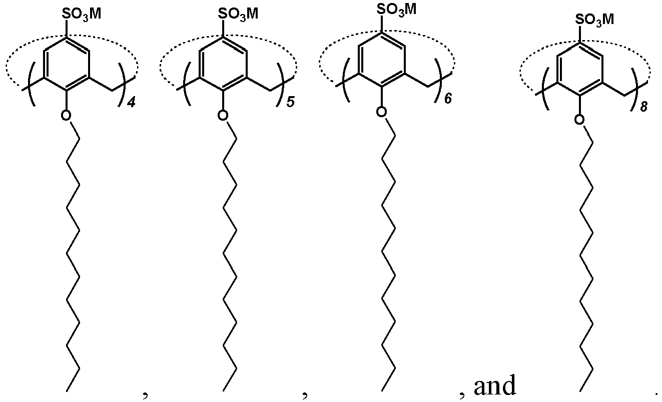

4. The skin-care product composition according to any of claims 1-3, wherein the skincare active substance is selected from one or more of:
active substances with cleansing or makeup removal functions, selected from butylene glycol, polyethylene glycol, dipropylene glycol, sodium dodecyl sulfate, sodium laureth sulfate, sodium acyl sulfate, decyl glucoside, cocamidopropyl betaine, amino acids, isopropyl myristate, isopropyl hexadecanoate, and triglyceride;
active substances with whitening functions, selected from vitamin C and derivatives thereof, phenylethyl resorcinol, niacinamide, tranexamic acid, arbutin, ellagic acid, p-dihydroxybenzene, kojic acid, glutathione, salicylic acid, tretinoin, and hydroquinone;
active substances with anti-aging functions, selected from vitamin A, proanthocyanidins, vitamin E, resveratrol, hexapeptide, palmitoyl tripeptide-5, bifida ferment lysate, astaxanthin, epidermal growth factor, glycolic acid, lactic acid, ubiquinone, caffeine, glycolic acid, lactic acid, vitamin C and derivatives thereof;
active substances with anti-inflammatory or anti-acne functions, selected from retinoic acid, α-hydroxy acid, salicylic acid, azelaic acid, and α-bisabolol;
active substances with moisturizing functions, selected from ceramides, sphingolipids, phospholipids, cholesterol, lecithin, squalane, hyaluronic acid, chondroitin sulfate, natural moisturizing factor, sorbitol, mannitol, glucose, trehalose, glycerin, pentylene glycol, butylene glycol, and provitamin B5;
active substances with sun protection functions, selected from benzophenone-3, octyl methoxycinnamate, ensulizole, PEG-25 p- aminobenzoic acid, ethylhexyl triazone, 2-ethylhexyl 2-cyano-3,3-diphenylacrylate, pentyl Padimate-O, polyacrylamide methylbenzylidene camphor, glyceryl p-aminobenzoate, diethylamino hydroxybenzoyl hexyl benzoate, camphor benzalkonium methosulfate, benzophenone-5, homosalate, ethylhexyl methoxycinnamate, isoamyl methoxycinnamate, and disodium phenyl dibenzimidazole tetrakisulfonate.

5. The skin-care product composition according to any of claims 1-4, wherein the molar ratio of the skincare active substance to the sulfonated calixarene compound is 1:(0.8-5.0), preferably 1:(0.9-3.0), more preferably 1:(1.0-1.5).

6. A pharmaceutical composition for treating skin inflammatory diseases, comprising at least one active substance with skin anti-inflammatory function and a sulfonated calixarene compound of formula (I): 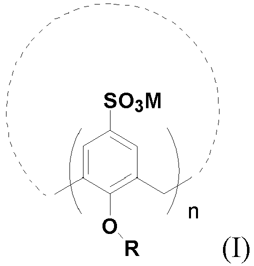 wherein,
n is an integer selected from 4 to 8,
M is independently selected from H, alkali metals, and alkaline earth metals,
R is independently selected from C₈₋₁₂ straight-chain alkyl.

7. The pharmaceutical composition according to claim 6, wherein the active substance with skin anti-inflammatory function is selected from one or more of: retinoic acid, α-hydroxy acid, salicylic acid, azelaic acid, and α-bisabolol.

8. The pharmaceutical composition according to claim 6 or 7, wherein M is at least one metal selected from the group consisting of Na, K, Mg, and Ca;
and/or wherein R is dodecyl.

9. The pharmaceutical composition according to any of claims 6-8, wherein the sulfonated calixarene compound of formula (I) is selected from:

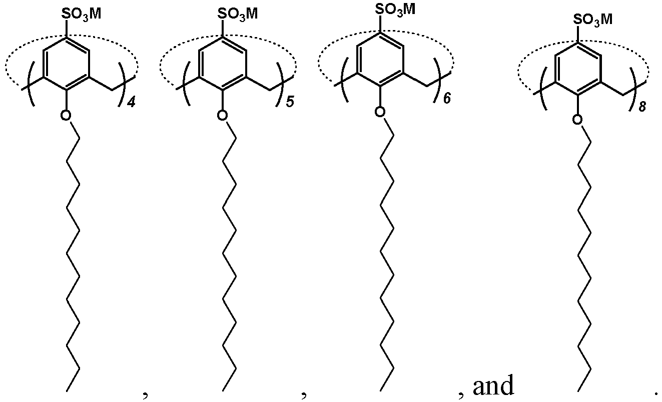

10. The pharmaceutical composition according to any of claims 6-9, wherein the skin inflammatory disease is at least one disease selected from: superficial scars, hypertrophic scars, keloids, lupus vulgaris, leprosy ulcers, tinea favosa, kerion, cutaneous cryptococcosis, impetigo, acne, and dermatitis;
or wherein the skin inflammatory disease is selected from at least one disease of the following: neurodermatitis selected from eczema, lichen, psoriasis, and pruritus; seborrheic dermatitis selected from psoriasis of the head and face, pityriasis rosea, and tinea corporis; and atopic dermatitis selected from erythema, papules, papulovesicles, exudative crusts, lichenification, skin scratches, and skin dryness.

11. The pharmaceutical composition according to any of claims 6-10, wherein the molar ratio of the active substance with skin anti-inflammatory function to the sulfonated calixarene compound is 1:(0.8-5.0), preferably 1:(0.9-3.0), more preferably 1:(1.0-1.5).

12. Use of the sulfonated calixarene compound of formula (I) for non-therapeutic skincare, 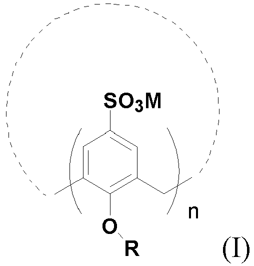 wherein,
n is an integer selected from 4 to 8,
M is independently selected from H, alkali metals, and alkaline earth metals,
R is independently selected from C₈₋₁₂ straight-chain alkyl.

13. A sulfonated calixarene compound of formula (I) for use in treating skin inflammatory diseases, 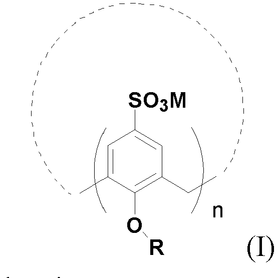 wherein,
n is an integer selected from 4 to 8,
M is independently selected from H, alkali metals, and alkaline earth metals,
R is independently selected from C₈₋₁₂ straight-chain alkyl.

14. The use of the compound according to claim 12, or the compound for use according to claim 13, wherein M is at least one metal selected from the group consisting of Na, K, Mg, and Ca; and/or
wherein R is dodecyl.

15. The use of the compound, or the compound for use, according to any of claims 12-14, wherein the sulfonated calixarene compound of formula (I) is selected from:

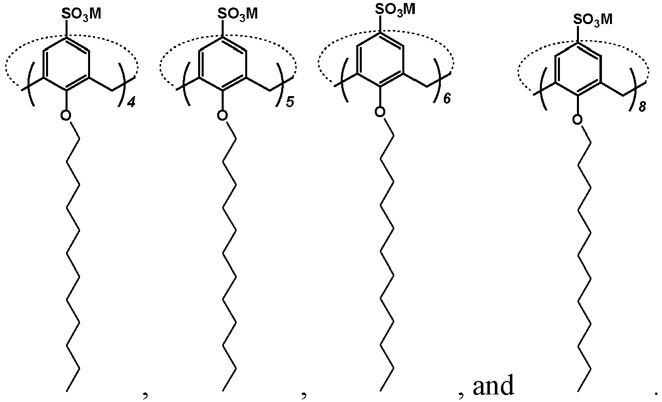

## Patentansprüche

1. Hautpflegeproduktzusammensetzung, umfassend mindestens einen Hautpflegewirkstoff und eine sulfonierte Calixarenverbindung von Formel (I): 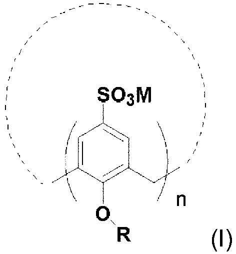 wobei
n eine ganze Zahl ist, die aus 4 bis 8 ausgewählt ist,
M unabhängig aus H, Alkalimetallen und Erdalkalimetallen ausgewählt ist,
R unabhängig aus geradkettigem C₈₋₁₂-Alkyl ausgewählt ist,
der mindestens eine Hautpflegewirkstoff aus Wirkstoffen ausgewählt ist, die mindestens eine der folgenden Funktionen aufweisen: Reinigung oder Make-up-Entfernung, Aufhellung, Alterungsschutz, Entzündungshemmung oder Anti-Akne, Feuchtigkeitsversorgung und Sonnenschutz.

2. Hautpflegeproduktzusammensetzung nach Anspruch 1, wobei M mindestens ein Metall ist, das aus der Gruppe ausgewählt ist, bestehend aus Na, K, Mg und Ca;
und/oder wobei R Dodecyl ist.

3. Hautpflegeproduktzusammensetzung nach Anspruch 1 oder 2, wobei die sulfonierte Calixarenverbindung von Formel (I) ausgewählt ist aus:

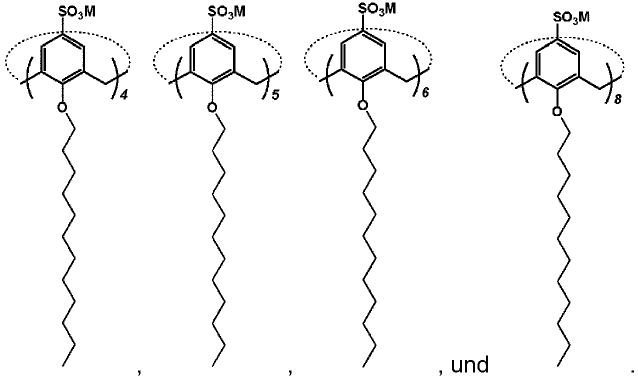

4. Hautpflegeproduktzusammensetzung nach einem der Ansprüche 1 bis 3, wobei der Hautpflegewirkstoff aus einem oder mehreren ausgewählt ist von:
Wirkstoffen mit Reinigungs- oder Make-up-Entfernungsfunktionen, ausgewählt aus Butylenglykol, Polyethylenglykol, Dipropylenglykol, Natriumdodecylsulfat, Natriumlaurethsulfat, Natriumacylsulfat, Decylglucosid, Cocamidopropylbetain, Aminosäuren, Isopropylmyristat, Isopropylhexadecanoat und Triglycerid;
Wirkstoffen mit Aufhellungsfunktionen, ausgewählt aus Vitamin C und Derivaten davon, Phenylethylresorcin, Niacinamid, Tranexamsäure, Arbutin, Ellagsäure, p-Dihydroxybenzol, Kojisäure, Glutathion, Salicylsäure, Tretinoin und Hydrochinon;
Wirkstoffen mit Alterungsschutzfunktionen, ausgewählt aus Vitamin A, Proanthocyanidinen, Vitamin E, Resveratrol, Hexapeptid, Palmitoyltripeptid-5, Bifidafermentlysat, Astaxanthin, epidermalem Wachstumsfaktor, Glykolsäure, Milchsäure, Ubichinon, Koffein, Glykolsäure, Milchsäure, Vitamin C und Derivaten davon;
Wirkstoffen mit entzündungshemmenden oder Anti-Akne-Funktionen, ausgewählt aus Retinsäure, α-Hydroxysäure, Salicylsäure, Azelainsäure und α-Bisabolol;
Wirkstoffen mit feuchtigkeitsspendenden Funktionen, ausgewählt aus Ceramiden, Sphingolipiden, Phospholipiden, Cholesterin, Lecithin, Squalan, Hyaluronsäure, Chondroitinsulfat, natürlichem Feuchtigkeitsfaktor, Sorbitol, Mannit, Glucose, Trehalose, Glycerin, Pentylenglykol, Butylenglykol und Provitamin B5;
Wirkstoffen mit Sonnenschutzfunktionen, ausgewählt aus Benzophenon-3, Octylmethoxycinnamat, Ensulizol, PEG-25 p- Aminobenzoesäure, Ethylhexyltriazon, 2-Ethylhexyl-2-cyano-3,3-diphenylacrylat, Pentylpadimat-O, Polyacrylamid-Methylbenzylidencampher, Glyceryl-p-aminobenzoat, Diethylaminohydroxybenzoylhexylbenzoat, Campherbenzalkoniummethosulfat, Benzophenon-5, Homosalat, Ethylhexylmethoxycinnamat, Isoamylmethoxycinnamat und Dinatriumphenyldibenzimidazoltetrasulfonat.

5. Hautpflegeproduktzusammensetzung nach einem der Ansprüche 1 bis 4, wobei das Molverhältnis des Hautpflegewirkstoffs zu der sulfonierten Calixarenverbindung 1 : (0,8-5,0), vorzugsweise 1 : (0,9-3,0), mehr bevorzugt 1 : (1,0-1,5) beträgt.

6. Pharmazeutische Zusammensetzung zum Behandeln von entzündlichen Hauterkrankungen, umfassend mindestens einen Wirkstoff mit hautentzündungshemmender Funktion und eine sulfonierte Calixarenverbindung von Formel (I): 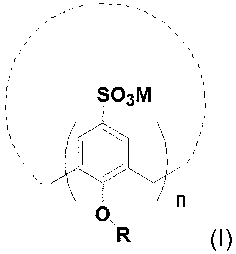 wobei
n eine ganze Zahl ist, die aus 4 bis 8 ausgewählt ist,
M unabhängig aus H, Alkalimetallen und Erdalkalimetallen ausgewählt ist,
R unabhängig aus geradkettigem C₈₋₁₂-Alkyl ausgewählt ist.

7. Pharmazeutische Zusammensetzung nach Anspruch 6, wobei der Wirkstoff mit hautentzündungshemmender Funktion aus einem oder mehreren ausgewählt ist von: Retinsäure, α-Hydroxysäure, Salicylsäure, Azelainsäure und α-Bisabolol.

8. Pharmazeutische Zusammensetzung nach Anspruch 6 oder 7, wobei M mindestens ein Metall ist, das aus der Gruppe ausgewählt ist, bestehend aus Na, K, Mg und Ca;
und/oder wobei R Dodecyl ist.

9. Pharmazeutische Zusammensetzung nach einem der Ansprüche 6 bis 8, wobei die sulfonierte Calixarenverbindung von Formel (I) ausgewählt ist aus:

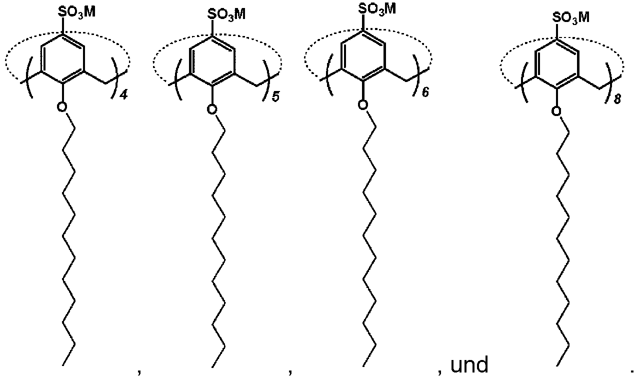

10. Pharmazeutische Zusammensetzung nach einem der Ansprüche 6 bis 9, wobei die entzündliche Hauterkrankung mindestens eine Erkrankung ist, die ausgewählt ist aus: oberflächlichen Narben, hypertrophen Narben, Keloiden, Lupus vulgaris, Leprageschwüren, Tinea favus, Kerion, Kryptokokkose der Haut, Impetigo, Akne und Dermatitis;
oder wobei die entzündliche Hauterkrankung aus mindestens einer der folgenden Erkrankungen ausgewählt ist: Neurodermitis, ausgewählt aus Ekzem, Lichen, Psoriasis und Pruritus; seborrhoischer Dermatitis, ausgewählt aus Psoriasis des Kopfes und des Gesichts, Pityriasis rosea und Tinea corporis; und atopischer Dermatitis, ausgewählt aus Erythema, Papeln, Papulovesikeln, Krusten mit Absonderung, Lichenifikation, Hautkratzern und Hauttrockenheit.

11. Pharmazeutische Zusammensetzung nach einem der Ansprüche 6 bis 10, wobei das Molverhältnis des Wirkstoffs mit hautentzündungshemmender Funktion zu der sulfonierten Calixarenverbindung 1 : (0,8-5,0), vorzugsweise 1 : (0,9-3,0), mehr bevorzugt 1 : (1,0-1,5) beträgt.

12. Verwendung der sulfonierten Calixarenverbindung von Formel (I) für eine nicht therapeutische Hautpflege, 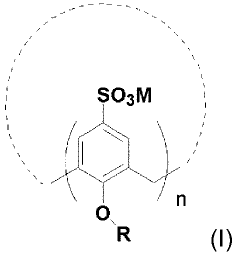 wobei
n eine ganze Zahl ist, die aus 4 bis 8 ausgewählt ist,
M unabhängig aus H, Alkalimetallen und Erdalkalimetallen ausgewählt ist,
R unabhängig aus geradkettigem C₈₋₁₂-Alkyl ausgewählt ist.

13. Sulfonierte Calixarenverbindung von Formel (I) zur Verwendung bei dem Behandeln von entzündlichen Hauterkrankungen, 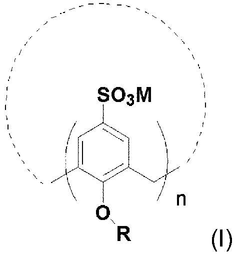 wobei
n eine ganze Zahl ist, die aus 4 bis 8 ausgewählt ist,
M unabhängig aus H, Alkalimetallen und Erdalkalimetallen ausgewählt ist,
R unabhängig aus geradkettigem C₈₋₁₂-Alkyl ausgewählt ist.

14. Verwendung der Verbindung nach Anspruch 12 oder Verbindung zur Verwendung nach Anspruch 13, wobei M mindestens ein Metall ist, das aus der Gruppe ausgewählt ist, bestehend aus Na, K, Mg und Ca; und/oder
wobei R Dodecyl ist.

15. Verwendung der Verbindung oder Verbindung zur Verwendung nach einem der Ansprüche 12 bis 14, wobei die sulfonierte Calixarenverbindung von Formel (I) ausgewählt ist aus:

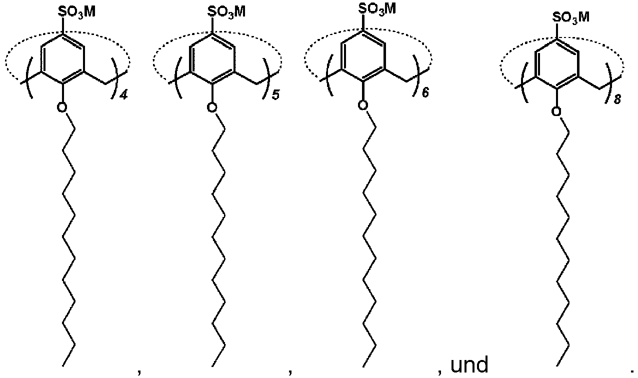

## Revendications

1. Composition de produit de soin de la peau, comprenant au moins une substance active de soin de la peau et un composé calixarène sulfoné de formule (I) : 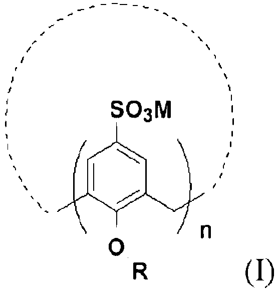 dans laquelle,
n est un nombre entier choisi de 4 à 8,
M est indépendamment choisi parmi H, les métaux alcalins et les métaux alcalino-terreux,
R est indépendamment choisi parmi un alkyle à chaîne droite en C₈₋₁₂,
l'au moins une substance active de soin de la peau est choisie parmi des substances actives qui présentent au moins l'une des fonctions suivantes : nettoyante ou démaquillante, blanchissante, anti-âge, anti-inflammatoire ou anti-acnéique, hydratante, et protégeant du soleil.

2. Composition de produit de soin de la peau selon la revendication 1, dans laquelle M est au moins un métal choisi dans le groupe constitué de Na, K, Mg et Ca ;
et/ou dans laquelle R est dodécyle.

3. Composition de produit de soin de la peau selon la revendication 1 ou 2, dans laquelle le composé calixarène sulfoné de formule (I) est choisi parmi :

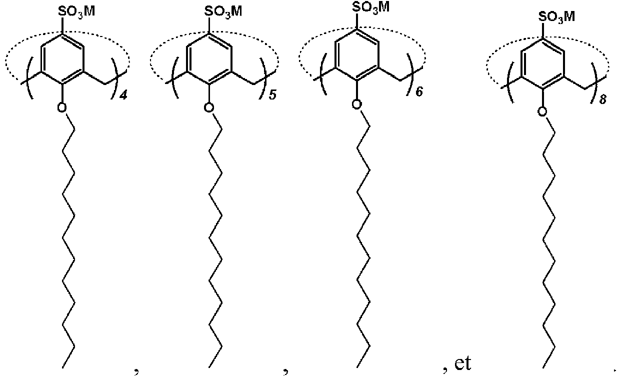

4. Composition de produit de soin de la peau selon l'une quelconque des revendications 1 à 3, dans laquelle la substance active de soin de la peau est choisie parmi un ou plusieurs de :
substances actives ayant des fonctions de nettoyage ou de démaquillage, choisies parmi butylène glycol, le glycol polyéthylénique, le dipropylèneglycol, le dodécyl sulfate de sodium, le laureth sulfate de sodium, l'acylsulfate de sodium, le décyl glucoside, la cocamidopropylbétaïne, les acides aminés, le myristate d'isopropyle, l'hexadécanoate d'isopropyle et un triglycéride ;
substances actives ayant des fonctions de blanchiment, choisies parmi la vitamine C et ses dérivés, le phényléthyl résorcinol, la niacinamide, l'acide tranexamique, l'arbutine, l'acide ellagique, le p-dihydroxybenzène, l'acide kojique, le glutathion, l'acide salicylique, la trétinoïne et l'hydroquinone ;
substances actives ayant des fonctions anti-âge, choisies parmi la vitamine A, les proanthocyanidines, la vitamine E, le resvératrol, l'hexapeptide, le palmitoyl tripeptide-5, le lysat de ferment de bifida, l'astaxanthine, le facteur de croissance épidermique, l'acide glycolique, l'acide lactique, l'ubiquinone, la caféine, l'acide glycolique, l'acide lactique, la vitamine C et leurs dérivés ;
substances actives ayant des fonctions anti-inflammatoires ou anti-acnéiques, choisies parmi l'acide rétinoïque, l'acide α-hydroxylé, l'acide salicylique, l'acide azélaïque et l'α-bisabolol ;
substances actives ayant des fonctions hydratantes, choisies parmi les céramides, les sphingolipides, les phospholipides, le cholestérol, la lécithine, le squalane, l'acide hyaluronique, le chondroïtine sulfate, un facteur naturel d'hydratation, le sorbitol, le mannitol, le glucose, le tréhalose, la glycérine, le pentylène glycol, le butylène glycol et la provitamine B5 ;
substances actives ayant des fonctions de protection solaire, choisies parmi benzophénone-3, méthoxycinnamate d'octyle, ensulizole, acide PEG-25 p-aminobenzoïque, éthylhexyl triazone, 2-Éthylhexyl 2-cyano-3,3-diphénylacrylate, pentyl Padimate-O , polyacrylamidométhyl benzylidène camphor, p-aminobenzoate de glycéryle, benzoate de diéthylamino hydroxybenzoyl hexyle, méthosulfate de benzalkonium de camphre , benzophénone-5, homosalate, éthylhexyl méthoxycinnamate, méthoxycinnamate d'isoamyle, et disodium phenyl dibenzimidazole tetrasulfonate.

5. Composition de produit de soin de la peau selon l'une quelconque des revendications 1 à 4, dans laquelle le rapport molaire de la substance active du soin de la peau au composé calixarène sulfoné est de 1:(0,8-5,0), de préférence de 1:(0,9-3,0), plus préférablement de 1:(1,0-1,5).

6. Composition pharmaceutique pour le traitement de maladies inflammatoires cutanées, comprenant au moins une substance active ayant une fonction anti-inflammatoire cutanée et un composé calixarène sulfoné de formule (I) : 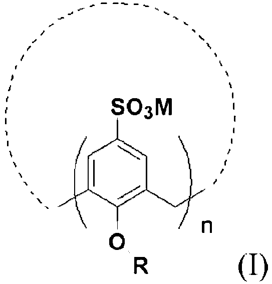 dans laquelle,
n est un nombre entier choisi de 4 à 8,
M est indépendamment choisi parmi H, les métaux alcalins et les métaux alcalino-terreux,
R est indépendamment choisi parmi un alkyle à chaîne droite en C₈₋₁₂.

7. Composition pharmaceutique selon la revendication 6, dans laquelle la substance active ayant une fonction anti-inflammatoire cutanée est choisie parmi un ou plusieurs parmi : l'acide rétinoïque, l'acide α-hydroxylé, l'acide salicylique, l'acide azélaïque et l'α-bisabolol.

8. Composition pharmaceutique selon la revendication 6 ou 7, dans laquelle M est au moins un métal choisi parmi le groupe constitué de Na, K, Mg et Ca ;
et/ou dans laquelle R est dodécyle.

9. Composition pharmaceutique selon l'une quelconque des revendications 6 à 8, dans laquelle le composé calixarène sulfoné de formule (I) est choisi parmi :

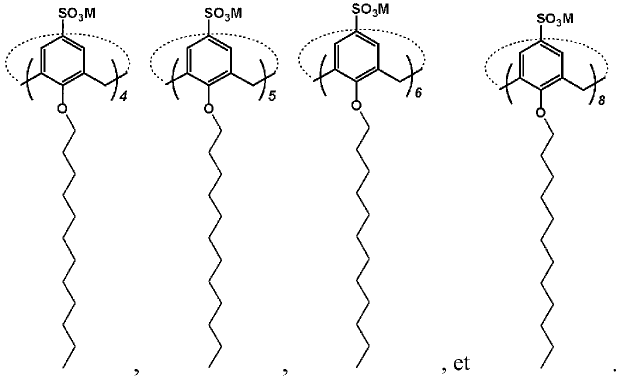

10. Composition pharmaceutique selon l'une quelconque des revendications 6 à 9, dans laquelle la maladie inflammatoire cutanée est au moins une maladie choisie parmi : les cicatrices superficielles, les cicatrices hypertrophiques, les chéloïdes, le lupus tuberculeux, les ulcères lépreux, la teigne favique, le kérion, la cryptococcose cutanée, l'impétigo, l'acné et la dermatite ;
ou dans laquelle la maladie inflammatoire cutanée est choisie parmi au moins une des maladies suivantes : la neurodermatite choisie parmi l'eczéma, le lichen, le psoriasis et le prurit ; la dermatite séborrhéique choisie parmi le psoriasis de la tête et du visage, le pityriasis rosé et le tinea corporis ; et la dermatite atopique choisie parmi l'érythème, les papules, les papulovésicules, les croûtes exsudatives, la lichénification, les écorchures cutanées et la sécheresse cutanée.

11. Composition pharmaceutique selon l'une quelconque des revendications 6 à 10, dans laquelle le rapport molaire de la substance active ayant une fonction anti-inflammatoire cutanée au composé calixarène sulfoné est de 1:(0,8-5,0), de préférence 1:(0,9- 3,0), plus préférablement 1:(1,0-1,5).

12. Utilisation du composé calixarène sulfoné de formule (I) pour le soin non thérapeutique de la peau, 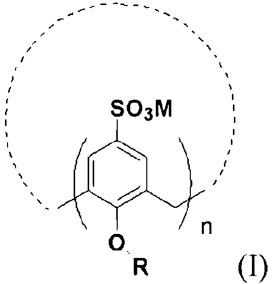 dans laquelle,
n est un nombre entier choisi de 4 à 8,
M est indépendamment choisi parmi H, les métaux alcalins et les métaux alcalino-terreux,
R est indépendamment choisi parmi un alkyle à chaîne droite en C₈₋₁₂.

13. Composé calixarène sulfoné de formule (I) pour une utilisation dans le traitement des maladies inflammatoires cutanées, 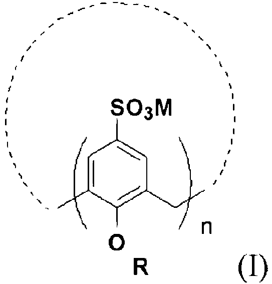 dans laquelle,
n est un nombre entier choisi de 4 à 8,
M est indépendamment choisi parmi H, les métaux alcalins et les métaux alcalino-terreux,
R est indépendamment choisi parmi un alkyle à chaîne droite en C₈₋₁₂.

14. Utilisation du composé selon la revendication 12, ou le composé pour une utilisation selon la revendication 13, dans laquelle M est au moins un métal choisi dans le groupe constitué par Na, K, Mg et Ca ; et/ou
dans laquelle R est dodécyle.

15. Utilisation du composé, ou le composé pour une utilisation, selon l'une quelconque des revendications 12 à 14, dans laquelle le composé calixarène sulfoné de formule (I) est choisi parmi :
